# EUROPEAN PATENT APPLICATION

(11) **EP 1 532 861 A1**
(43) Date of publication of application: **25.05.2005**
(21) Application number: 03723398.8
(22) Date of filing: 26.05.2003
(51) Int. Cl.: A01K 67/027, C12N 9/64, C12N 15/12, C12N 15/63

(54) **CATHEPSIN-ASSOCIAGED GENETICALLY MODIFIED NONHUMAN MAMMAL**

(30) Priority: 28.05.2002 JP 2002153641
(71) Applicant: Kyushu TLO Company, Limited, Fukuoka-shi, Fukuoka 812-8581 (JP)
(72) Inventor: Yamamoto, Kenji, Fukuoka-shi, Fukuoka 811-0215 (JP); Nakayama, Keiichi, Fukuoka-shi, Fukuoka 812-0053 (JP)
(74) Representative: McCallum, Graeme David
(86) International application number: PCT/JP2003/006507
(87) International publication number: WO 2003/099002

(57) **Abstract**

Disclosed are a cathepsin E gene and a non-human mammalian animal with the cathepsin E-associated gene altered. The cathepsin E-associated gene and the DNA fragment thereof have each a base sequence or a partial amino acid sequence as identified by SEQ ID #1. The cathepsin E-associated gene-altered non-human mammalian animal according to the present invention, such as a cathepsin E-associated gene-altered mouse or the like, has the functions of the cathepsin E-associated gene by performing homologous recombination with a targeting vector having two homologous recombination regions, the first homologous recombination region being composed of a DNA fragment of approximately 1.2 kbp present on the 5'-upstream side of exon 1 and the second homologous recombination region being composed of a DNA fragment of approximately 7.0 kbp present on the 3'-downstream side of exon 4. The cathepsin E-associated gene-altered non-human mammalian animals according to the present invention can be used effectively for clarification of allergic diseases such as atopic dermatitis and so on as well as their disease conditions, and they are expected to be utilized as an experimental animal model for use with experiments on learning disabilities or memory impairments or acceleration of fighting episodes. Moreover, the cathepsin E-associated gene-altered non-human mammalian animal of the present invention is expected to be useful for the elucidation of the physiological functions about stress because it has a very high sensitiveness against stress.

## Description

### Technical Field

The present invention relates to a cathepsin gene-altered non-human mammalian animal and, more particularly, to a gene-altered non-human mammalian animal particularly with a cathepsin E-associated gene deleted, to a method for the production thereof, to a targeting vector for the production thereof, and to a method of the construction thereof as well as to a method for using the same.

### Background Technology

Aspartic proteinases distribute widely from a higher animal to a microorganism and have important biological functions including intracellular and extracellular protein metabolisms, their processing and so on. As one of such aspartic proteinases of a higher animal, cathepsin D and cathepsin E are present in an intracellular endosome/lysosome system of the higher animal. As aspartic proteinases are involved in the intracellular and extracellular protein metabolisms and processing thereof, a variation with the level of their activities has been considered to lead to various disease conditions including, for example, abnormality in blood pressure, a gastric ulcer, oncogenesis, and so on.

Among those cathepsins, cathepsin D is an intracellular aspartic proteinase that is most well known in the pepsin family and distributes widely in almost all animal cells. Further, it is a lysosome enzyme that is present in the largest amount and that is known to cleave a hydrophobic peptide linkage including Phe-Phe, Phe-Tyr, Tyr-Leu, Leu-Tyr, and the like.

The function of the cathepsin D is considered to be involved in a non-specific terminal degradation of proteins within the lysosome system due to its wide distribution in tissues and a mode of the non-specific degradation upon using a proteinaceous substance such as hemoglobin, or so on. It is suggested, however, that the cathepsin D plays an important physiological function due to the results of experiments using cathepsin D knocked-out mice that it decomposes such a protein as involving in the growth and multiplication of cells in a limited way rather than a non-specific terminal degradation of proteins.

It has also been reported that cathepsin D has the ability to produce an angiogenesis-repressing factor as a novel function. This function is such that cathepsin D secreted outside the cells from the human prostate cancer cells produces the angiogenesis-repressing factor, angiostatin, from plasminogen and represses the growth or metastasis of tumor cells. It is further known that the angiogenesis is of great significance from the physiological point of view in terms of the formation of the fetus and placenta, the postnatal growth of tissues, the cure of injury, and so on, while it is associated closely with the growth of solid tumors and the development of morbidity including, for example, rheumatoid arthritis, ophthalmic diseases represented by diabetic retinitis, and so on. Studies toward a clinical application of proteinases producing *in vivo* the endogenous angiogenesis-repressing factors are expected to a great extent, therefore, because an angiogenesis repressor has the capability of resulting in a therapeutic agent effective against intractable diseases accompanying the angiogenesis, such as cancers, articular rheumatism, etc.

On the other hand, cathepsin E is an intracellular aspartic proteinase, unlike cathepsin D, which distributes in limited areas including the epithelia of the digestive tract, such as stomach and intestine, lymphoid tissues, tissues of the urogenital system, blood tissues and skin. In particular, the stomach is the organ that contains cathepsin E in the largest amount among all tissues and in the amount larger than cathepsin D. The intracellular localization of cathepsin E is obviously different from that of cathepsin D and the cathepsin E has specificity to tissues and cells. Cathepsin E is localized in cell membranes of the erythrocytes, osteoclasts and proximal urinary cells, etc., in the endosome/lysosome system of microglia or macrophages, and in the endoplasmic reticulum and Golgi apparatus of many other peripheral tissues. Studies so far conducted reveal that cathepsin E can express its proteinase activity as a mature structure only when it is localized in the endosome/lysosome system of almost all cells except erythrocytes. Further, for the microglia and macrophages, when interferon-gamma or a lipopolysaccharide, etc. is activated by the addition of some stimulation to cells, cathepsin E demonstrates a remarkable increase at the mRNA level as well as at the protein level. This pnehomenon suggests that cathepsin E is closely involved in these cell functions.

Moreover, cathepsin E is little detectable in the neurocytes of juvenile rats, however, its presence can be obviously confirmed in the neurocytes of aged rats which are accumulated with lipofuscin or C-terminal fragments of APP (amyloid precursor protein). Furthermore, in the brain of a rat with the forebrain ischemia or with kainic acid administered thereto, a remarkable increase in the expression of cathepsin E can be recognized in the neurocytes undergoing denaturation depending upon the weak site against these stimulation and in the microglia accumulated and activated in response to these stimulation. These results suggest that cathepsin E plays an important role in the process of executing the death of neurons. In order to clarify the physiological functions of cathepsin E having such unique functions, there is a demand for the development of gene-altered non-human mammalian animals, such as knocked-out mice and so on.

As described above, the successful development of such gene-altered non-human mammalian animals including knocked-out mice, etc., particularly with cathepsin E-associated genes knocked-out, can directly explicate the physiological functions of cathepsin E as well as contribute greatly to the elucidation of various disease conditions with the cathepsin E-associated genes involved therein and studies on the therapy method of such diseases. Moreover, such gene-altered non-human mammalian animals are extremely useful as experimental animal models because they have a definite genetic background.

Generally, it is desired to use animals as experimental animals, which have established lines, that is, which are genetically homologous. As the homologous animals which have been established as lines have been investigated in detail for their genetic backgrounds, studies using such experimental animals having the known genetic backgrounds can readily determine only the effects produced by the treatment applied to the experimental animals. On the other hand, a hybrid animal has various genes in a heterogeneous state so that it is difficult to distinguish whether the effect resulting from the treatment applied to the hybrid animal is derived only from the treatment or from the genetic background. It is expected, therefore, that the gene-altered non-human mammalian animal with the cathepsin E gene deleted can directly clarify the physiological functions of cathepsin E involving various physiological functions as well as plays great roles in making clear various diseases and disease conditions, in which the cathepsin E-associated genes are involved, and performing studies on the therapy method of such diseases.

### Disclosure of the Invention

As a result of extensive review and studies on the production of gene-altered non-human mammalian animals with cathepsin E-associated genes deleted, the present inventors have now determined the base sequence and the amino acid sequence of a cathepsin E-associated gene and, at the same time, found that a knocked-out mouse with a cathepsin E-associated gene deleted can be produced by means of the gene targeting method by homologous recombination using a portion of the cathepsin E-associated gene. The present invention is based on this determination and finding.

Therefore, the present invention has one object to provide a gene-altered non-human mammalian animal with a cathepsin E-associated gene deleted.

The gene-altered non-human mammalian animal with such a cathepsin E-associated gene altered can be produced using the gene targeting method by the homologous recombination of the cathepsin E-associated gene, and the resulting gene-altered non-human mammalian animal with the cathepsin E-associated gene deleted is found extremely useful for the elucidation of various disease conditions in which the cathepsin E-associated gene is involved and for studies on the method for therapy of such diseases as well as can be utilized as an experimental animal model having a definite genetic background.

As it is suggested that cathepsin E plays a significant role in the process of executing the death of neurons, the gene-altered non-human mammalian animal also has an extremely great role in making clear the physiological mechanisms of cathepsin E having such a unique function.

Another object of the present invention is to provide a cathepsin E-associated gene and a DNA fragment of the cathepsin E-associated gene.

A more full base sequence and amino acid sequence of the cathepsin E-associated gene provided by the present invention assist in the elucidation of the physiological functions of the cathepsin E-associated gene as well as play a great role in developing means and tools useful for the diagnosis and the method for therapy of disease conditions and diseases in which the cathepsin E-associated gene is involved.

The present invention has another object to provide a targeting vector to be constructed for performing homologous recombination by the insertion of a DNA fragment of the cathepsin E-associated gene.

The targeting vector according to the present invention is the one that suppresses the expression of a desired gene by causing homologous recombination to occur through the homologously recombined DNA fragment integrated into the vector and then demonstrates no function of the involved gene. Therefore, the targeting vector is a core element for the production of the gene-altered non-human mammalian animal with the desired function deleted or altered so that the configuration of the targeting vector is one of the major objects of the present invention.

The present invention has also another object to provide a plasmid functioning as the targeting vector.

In accordance with the present invention, the appropriate selection of such a plasmid enables playing a role as the targeting vector in a more efficient fashion and improves the adaptability of the plasmid with an embryonic stem cell (ES cell) into which the plasmid is to be inserted, causing homologous recombination to occur at a higher rate.

A still another object of the present invention is to provide a method for the production of a gene-altered non-human mammalian animal with the cathepsin E-associated gene deleted.

The gene-altered non-human mammalian animal with the deleted cathepsin E-associated gene produced by the method according to the present invention is expected to contribute greatly to the elucidation of various disease conditions in which the cathepsin E-associated gene is involved, and to studies on the method for therapy of such diseases as well as it is extremely useful as an experimental animal model because its genetic background is distinct, as described above.

The present invention also has an object to provide a method for the construction of the targeting vector. The provision of the appropriate method for the construction of the targeting vector having the very significant role as described above is also greatly significant for practicing the invention. Therefore, the construction method of the targeting vector according to the present invention enables constructing the targeting vector in an appropriate and efficient way.

Moreover, the present invention provides a method for using the gene-altered non-human mammalian animal according to the present invention for useful purposes, which is produced by the production method according to the present invention.

The gene-altered non-human mammalian animal according to the present invention is expected as being capable of being used as a standard experimental animal model for allergy and atopic dermatitis-like skin lesions because it can demonstrate very high sensitiveness to allergy and atopic dermatitis-like skin lesions. Therefore, the gene-altered non-human mammalian animal is expected to play an important role in elucidating the mechanism of expression of hardly curable diseases including, for example, allergy and atopic dermatitis-like skin lesions, eventually resulting in the great contribution to the diagnosis and therapy of the disease conditions of such diseases.

The present invention has a still another object to provide a method for using the cathepsin E-associated gene-altered non-human mammalian animal according to the present invention for diagnosis of learning disabilities or memory impairments, diagnosis of the acceleration of fighting episodes, and diagnosis of endurance against stress. As the cathepsin E-associated gene-altered non-human mammalian animal according to the present invention can express learning disabilities or memory impairments, thereby demonstrating specific symptoms similar to human's Alzheimer-like symptoms or demensia-like symptoms, it is expected to contribute to the elucidation of the expression mechanism as well as to assist in studying causes of human's Alzheimer's disease and diagnosing and treating the diseases.

Similarly, the gene-altered non-human mammalian animal according to the present invention may exhibit the acceleration of fighting episodes or demonstrate extremely high sensitiveness to stress, so that it is expected that it will play a significant role in throwing light upon causes of the functions of the acceleration of the fighting episodes and the expression thereof or upon elucidation of the physiological functions of sensitiveness to stress and the expression thereof. As a result, the gene-altered non-human mammalian animal according to the present invention will be able to contribute to the development of methods for diagnosis and therapy including, but being not limited to, diagnosing or curing the acceleration of the fighting episodes or stress.

In order to achieve the objects as described above, in one aspect, the present invention provides a gene-altered non-human mammalian animal with a cathepsin E-associated gene altered by performing homologous recombination of the cathepsin E-associated gene. In a preferred embodiment of the aspect of the present invention, there is provided a rodent with the cathepsin E-associated gene deleted as the gene-altered non-human mammalian animal, particularly a knocked-out mouse.

The cathepsin E-associated gene-altered non-human mammalian animal according to the present invention can realize an extremely great role in making clear the physiological functions of cathepsin E having unique functions as described above. As the physiological functions of the cathepsin E would have been made clear, this elucidation will be capable of contributing greatly to studies on solutions to various disease conditions in which the cathepsin E-associated genes are involved, as well as the methods for diagnosis and therapy for such diseases. Moreover, the gene-altered non-human mammalian animal according to the present invention will be extremely useful as an experimental animal model because it is found to have a distinct genetic background.

It is to be noted herein that the term "gene-altered" as used in this description is intended to mean an event in which other gene or a fragment thereof is subjected to homogenous recombination means including, for example, modifications, deletions, substitutions or insertions or the like, thereby resulting in deleting or making deficient or modifying the gene of a non-human mammal as a host, particularly the gene originally existing in a mammal and causing no expressing the gene and enabling demonstrating no physiological functions thereof.

Therefore, the term "gene-altered non-human mammalian animal" as used herein is intended to mean a non-human mammal with the expression of the cathepsin E-associated gene suppressed and consequently with the functions thereof made deficient by deleting, making deficient or modifying, etc. the gene of the non-human mammal as a host, in particular the gene original in the mammal by performing genetic genetic alteration means including, but not being limited to, modifications, deletions, substitutions or insertions for another gene or a fragment thereof.

As used herein, the term "cathepsin E-associated gene-altered non-human mammalian animal" and related terms, accordingly, are intended to mean a non-human mammalian animal with the cathepsin E-associated gene altered, in which the expression of the cathepsin E-associated gene is suppressed and consequently the functions thereof are made deficient by performing genetic alteration means including, for example, modifications, deletions, substitutions or insertions, etc.

It should also be noted herein that the various means such as "modifications", "deletions", "insertions" and "substitutions ", as used herein for genetic alteration means, are not used in any respect in limited terms or with intentions to clearly distinguish each means from others and are used solely to alter the cathepsin E-associated gene by either of such genetic alteration means, resulting as a consequence in the suppression of the expression of the cathepsin E-associated gene and the deficiency of the functions thereof. It can further be understood herein that the above means is used generally as having the following meanings.

Generally, the "modifications" of a genomic DNA refer to the event in which one or more bases of a genomic DNA fragment of the cathepsin E-associated gene is or are modified to thereby make the resulting expression product of the cathepsin E-associated gene incapable to work as a cathepsin E-associated molecule; the "deletions" refer to the event in which a portion or all of a genomic DNA fragment of the cathepsin E-associated gene is deleted to thereby make the resulting expression product of the cathepsin E-associated gene incapable to work as a cathepsin E-associated molecule or to allow it to become absent; the "substitutions" refer to the event in which one or more bases of a genomic DNA fragment of the cathepsin E-associated gene is or are replaced with another sequence not relating to the cathepsin E-associated gene to thereby make the resulting expression product of the cathepsin E-associated gene incapable to work as a cathepsin E-associated molecule or to allow it to become absent; and the "insertions" refer to the event in which a DNA having another sequence different from the cathepsin E-associated gene is inserted into one or more bases of a genomic DNA fragment of the cathepsin E-associated gene to thereby make the resulting expression product of the cathepsin E-associated gene with the inserted other DNA incapable to work as a cathepsin E-associated molecule.

Moreover, the genetic alteration means such as, for example, modifications, deletions, substitutions and insertions, may be used singly or in combination of two or more means simultaneously for the cathepsin E-associated gene. In addition, the cathepsin E-associated gene may be simultaneously subjected to alterations against one or more genes thereof.

In another mode of the present invention, there is provided a cathepsin E-associated gene having a particular base sequence as defined by SEQ ID #1 of SEQUENCE LISTING or a partial amino sequence thereof or a DNA fragment containing a partial sequence thereof. Further, as used herein, the partial sequence of the cathepsin E-associated gene is intended to mean an arbitrary partial sequence selected from the region from a transcriptional control region of the cathepsin E-associated gene to a translational termination codon.

In a preferred embodiment of the another mode as described above, the present invention provides the cathepsin E-associated gene or a DNA fragment containing a partial sequence thereof, which has homologous recombination regions at two sites working as an object of homologous recombination. Further, in a preferred embodiment, the present invention provides the cathepsin E-associated gene or a DNA fragment containing a partial sequence thereof, which contains two sites of the homogenous recombination regions comprising a first homogenous recombination region existing on the 5'-upstream side and a second homogenous recombination region existing on the 3'-downstream side.

Moreover, the present invention in a preferred embodiment provides the cathepsin E-associated gene or the DNA fragment containing a partial sequence thereof, in which the first homologous recombination region is composed of a DNA fragment of approximately 1.2 kbp located at the position upstream of exon 1 of the cathepsin E-associated gene and it is located in a region between a first cleavage site to be cleaved with a restriction enzyme *StuI* and a second cleavage site to be cleaved with a restriction enzyme *HindIII*.

In addition, in a preferred embodiment, the present invention provides the cathepsin E-associated gene or the DNA fragment thereof, in which the first homologous recombination region is composed of the DNA fragment having a base sequence ranging from the base of base number 1,438 to the base of base number 2,656 of the base sequence identified as SEQ ID #1.

Further, in a preferred embodiment, the present invention provides the cathepsin E-associated gene or the DNA fragment containing a partial sequence thereof, in which the second homogenous recombination region is composed of a DNA fragment of approximately 7.0 kbp located at the position downstream of exon 4 of the cathepsin E-associated gene and located in a region between the position upstream by 76 bp from a third cleavage site to be cleaved with a restriction enzyme *ScaI* and the position downstream by 52 bp from a fourth cleavage site to be cleaved with a restriction enzyme *HpaI*.

Furthermore, in a preferred embodiment, the present invention provides the DNA fragment in which the second homologous recombination region is composed of a base sequence ranging from the base of base number 6,417 to the base of base number 13,548.

In accordance with the present invention, the provision of the cathepsin E-associated gene or the DNA fragment thereof, which have the definite configuration of the DNA fragment, can achieve a great role in clarifying the physiological mechanisms of cathepsin E with unique functions and assist to a great extent in contributing to the elucidation of various disease conditions in which the cathepsin E-associated gene is involved and to the development of methods for diagnosis and therapy of various diseases relating to the cathepsin E-associated gene. Moreover, the cathepsin E-associated gene and the DNA fragment thereof according to the present invention are very useful for the production of the cathepsin E-associated gene-altered non-human mammalian animals as experimental animal models due to their definite genetic background.

In another aspect, the present invention provides a targeting vector in which both of the DNA fragment of the first homologous recombination region and the DNA fragment of the second homologous recombination region are inserted thereinto. For the targeting vector in a preferred embodiment of the present invention, a base sequence of the DNA fragment of the first homologous recombination region is constructed to have a sequence length shorter than that of the DNA fragment of the second homologous recombination region.

In a preferred embodiment, the present invention further provides the targeting vector in which the DNA fragment of the first homologous recombination region is preferably linked to the 5'-side of a first DNA region containing a positive selection marker gene with which a promoter or the like, preferably PGK promoter, is coupled and the DNA fragment of the second homologous recombination region is preferably linked between the 3'-side of the first DNA region and the 5'-side of a second DNA region containing a negative selection marker gene with which a promoter or the like, preferably PGK promoter, is coupled.

In a preferred embodiment, the present invention further provides the targeting vector in which the positive selection marker gene is neomycin transferase gene and the negative selection marker gene is thymidine kinase gene.

In a preferred embodiment, there is further provided the targeting vector having the DNA fragment of the second homologous recombination region containing exon 5 and exon 6. Moreover, as a still further preferred embodiment, the present invention provides the targeting vector in which the DNA fragment of the first homologous recombination region and the DNA fragment of the second homologous recombination region are inserted into given locations of a plasmid.

As a still another aspect, the present invention provides a method for the production of a gene-altered non-human mammalian animal with the cathepsin E-associated gene deleted by subjecting the cathepsin E-associated gene to homogenous recombination. In a preferred embodiment, the method according to the present invention provides a mouse with the cathepsin E-associated gene deleted. Further, in a preferred embodiment, the present invention provides the method for the production of the gene-altered non-human mammalian animal by performing homogenous recombination using the targeting vector.

As a still another aspect, the present invention provides a method for the production of the gene-altered non-human mammalian animal, which comprises the cloning step of collecting a genomic clone of the cathepsin E-associated gene; the targeting vector-constructing step of constructing the targeting vector; the homogenous recombination step of obtaining a homologous recombinant by inserting the targeting vector into an embryonic stem cell (ES cell); the ES cell-injecting step of injecting the ES cell with the cathepsin E-associated gene deleted into the blastula; the chimera-breeding step of breeding a chimeric non-human mammalian animal; and the breeding step of breeding a gene-deleted non-human mammalian animal. In a preferred embodiment according to the present invention, there is provided a mouse with the cathepsin E-associated gene deleted as a preferred object of the gene-altered non-human mammalian animal.

In another preferred embodiment, the present invention provides the method for the production of the gene-altered non-human mammalian animal which comprises producing the gene-altered non-human mammalian animal by means of gene targeting method.

In a still further preferred embodiment, there is provided the method for the production of the gene-altered non-human mammalian animal, which comprises collecting the genomic clone from the cathepsin E-associated gene having the base sequence identified by SEQ ID #1 or the DNA fragment thereof. In a further preferred embodiment, there is also provided a probe to be used for the cloning step, which comprises a genomic DNA or cDNA isolated from an animal equal in species to the gene-altered non-human mammalian animal to be used for the present invention.

As a still further preferred embodiment, the present invention provides the method for the production of the gene-altered non-human mammalian animal according to the present invention, which is directed to the homogenous recombination step comprising injecting the targeting vector into a pluripotent cell including, but being not limited to, the embryonic stem cell (ES cell) by means of electroporation.

In a still further preferred embodiment of the present invention, the method for the production of the gene-altered non-human mammalian animal contains the homologous recombinant-screening step which comprises subjecting the resultant homologous recombinant to double screening using the positive selection marker gene and the negative selection marker gene. In a still further preferred embodiment, neomycin resistant gene is used for the positive selection marker gene, and thymidine kinase gene is used for the negative selection marker gene.

As a still further preferred embodiment of the present invention, the method for the production of the gene-altered non-human mammalian animal further contains the ES cell-injecting step which comprises injecting the ES cell with the cathepsin E-associated gene deleted into the blastula by means of the microinjection method. In a still further preferred embodiment, the blastula may include, but is not limited to, blastocyst or the like.

In a still further preferred embodiment of the present invention, the method for the production of the gene-altered non-human mammalian animal is provided, in which the step for breeding the gene-altered non-human mammalian animal with the cathepsin E-associated gene deleted comprises breeding a heterozygous non-human mammalian animal by mating the chimeric non-human mammalian animal bred in the chimera-breeding step. In a still further preferred embodiment, the method further comprises a step for confirming a heterozygote of the cathepsin E gene from DNA of the chimeric non-human mammalian animal bred in the chimera-breeding step by PCR method.

As a still another aspect, the present invention provides a method for constructing the targeting vector for the production of the cathepsin E-associated gene-altered non-human mammalian animal by subjecting the cathepsin E-associated gene to homogenous recombination with the targeting vector. This construction method allows the construction of the targeting vector which has the configuration as described above and which is appropriate for the production of the non-human mammalian animal with the cathepsin E-associated gene altered.

As a still another aspect, the present invention provides a method for using the cathepsin E-associated gene-altered non-human mammalian animal for diagnosis of diseases including allergy, atopic dermatitis-like skin lesions, etc. or diagnosis of learning disabilities or memory impairments or acceleration of fighting episodes or stress endurance, or the like. For the gene-altered non-human mammalian animals provided by the present invention, the cathepsin E-associated gene is fully or substantially fully deleted so that it can be utilized for elucidating diseases or disease conditions in which the cathepsin E-associated gene is considered to be involved, thereby capable of being applied to diagnosis and curing of such diseases.

### Detailed Description of the Accompanying Drawings

Fig. 1 is a gene map showing a cathepsin E-associated gene.
Fig. 2 is a schematic diagram for explaining the construction of a targeting vector.
Fig. 3 is an electrophoresis diagram showing the result of Southern blot analysis.
Fig. 4 is an electrophoresis diagram showing the result of PCR method.
Fig. 5 is a graph showing assessment scores of observation results for each disease case of experimental contact dermatitis by hapten pasting.
Fig. 6 is a diagram showing the results of histopathological analysis of mice with cathepsin E-associated gene deleted and control mice for their skins, 15 weeks of age, with atopy-like dermatitis.
Fig. 7 is a diagram showing the results of histopathological analysis for skins of experimental mice with experimental contact dermatitis by pasting with hapten for a period of 5 month.
Fig. 8 is a diagram showing the results of measurements for electrical stimulation against mice with cathepsin E-associated gene deleted and control mice by measuring the time required for moving from the light chamber to the dark chamber as a learning acquisition step by electrical stimulation (stimulation interval: 1 second; stimulation time: 10 seconds) as a step-through latency.
Fig. 9 is a diagram showing the results of measurements for start time (A), lasting time (B) and frequency (C) of occurrences of fighting episodes (a confronting episode against each other by rising, a biting episode, crying episode, etc.) of cathepsin E-associated gene deleted mice and control mice.

### Best Modes for carrying out the Invention

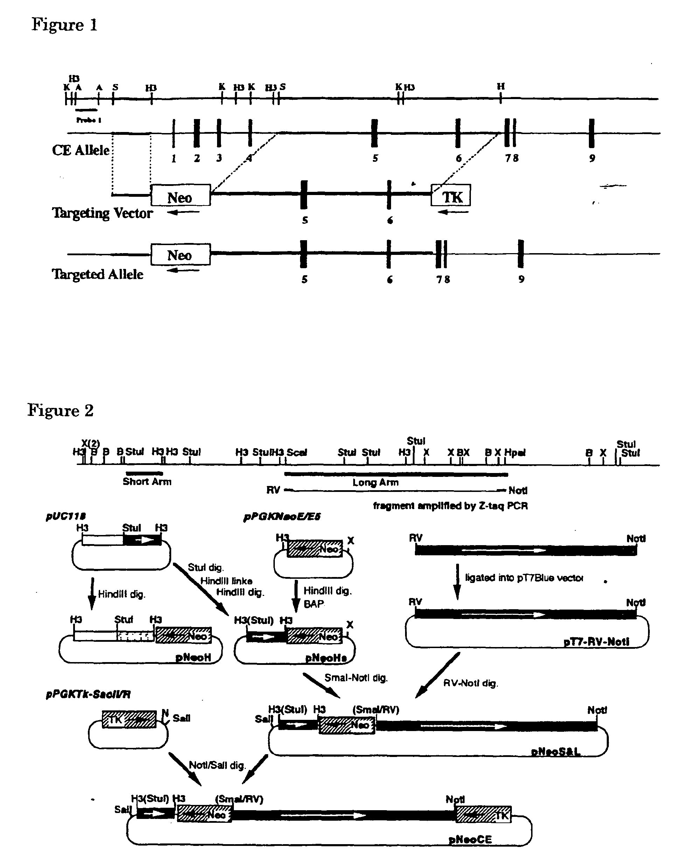

The cathepsin E-associated gene according to the present invention contains nine exons from exon 1 to exon 9, inclusive, as shown in Fig. 1. Also, the cathepsin E-associated gene and a DNA fragment thereof possess each one cleavage site to be cleaved with a restriction enzyme *KpnI* and with a restriction enzyme *StuI* as well as two cleavage sites each to be cleaved with a restriction enzyme *HindIII* and a restriction enzyme *AvaII* in a region on a 5'-upstream side of exon 1 thereof. Further, a region of each of exon 3 and exon 4 contains each one cleavage site to be cleaved with a restriction enzyme *KpnI*, and a region between exon 3 and exon 4 contains a cleavage site to be cleaved with a restriction enzyme *HindIII*. Moreover, a region between the 3'-downstream side of exon 4 and exon 5 contains one cleavage site to be cleaved with a restriction enzyme *HindIII* and three cleavage sites each to be cleaved with restriction enzyme *StuI*. In a region between exon 5 and exon 6, there further exists one cleavage site each to be cleaved with restriction enzymes *KpnI* and *HindIII*. In addition, a region between exon 6 and exon 7 contains one cleavage site to be cleaved with *HpaI*.

Further, the cathepsin E-associated gene or the DNA fragment thereof according to the present invention has two homogenous recombination regions. Of the two homogenous recombination regions, the homogenous recombination region existing on the 5'-upstream side is referred to hereinafter as a first homologous recombination region and the homogenous recombination region existing on the 3'-downstream side is referred to hereinafter as a second homologous recombination region.

The first homogenous recombination region exists on the 5'-upstream side of exon 1 and corresponds to a region existing between the *StuI* cleavage site and the second *HindIII* cleavage site. The second homogenous recombination region corresponds to a region existing between the position upstream by 76 bp from the *ScaI* cleavage site located between exon 4 and exon 5 and the position downstream by 52 bp from the *HpaI* cleavage site on the 5'-upstream side of exon 7. Therefore, the second homologous recombination region contains a portion of exon 5 and exon 6.

Alternatively, the cathepsin E-associated gene or the DNA fragment thereof according to the present invention has a base sequence and a partial amino acid sequence, each identified by SEQ ID #1 of SEQUENCE LISTING. More specifically, the DNA fragment of the first homologous recombination region of the cathepsin E-associated gene according to the present invention has a base sequence in the range from the base of base number 1,435 to the base of base number 2,661.

Therefore, the first homologous recombination region is composed of a base sequence of approximately 1.2 bp upstream of exon 1, and the second homologous recombination region is composed of a base sequence of approximately 7.0 bp downstream of exon 4.

It is to be noted herein that amino acids as used herein are represented by a three-letter abbreviation notation method. In the following description, the three-letter abbreviations have the following meanings: Ala stands for alanine; Arg for arginine; Asn for asparagine; Asp for aspartic acid; Cys for cysteine; Glu for glutamic acid; Gln for glutamine; Gly for glycine; His for histidine; Ile for isoleucine; Leu for leucine; Lys for lysine; Met for methionine; Phe for phenylalanine; Pro for proline; Ser for serine; Thr for threonine; Trp for tryptophan; Tyr for tyrosine; and Val for valine.

A description will be given hereinafter in more details regarding the gene-altered non-human mammalian animal according to the present invention by way of embodiments. It is also understood herein that the present invention is not interpreted in any respect to be limited by the following description and that such embodiments are described solely for illustrations of specific examples of the present invention.

The gene-altered non-human mammalian animal with the cathepsin E-associated gene deleted according to the present invention comprises a non-human mammal, particularly with the function of the cathepsin E-associated gene, deleted from the chromosome and with the integral gene of the non-human mammal encoding the cathepsin E-associated gene altered and consequently inactivated, thereby resulting in a lost of the expression mechanisms of the cathepsin E-associated gene.

In accordance with the present invention, specifically, the gene-altered non-human mammalian animal with the cathepsin E-associated gene deleted is directed to a mouse with the gene altered in the manner as described above by genetic engineering means known to the art. Also, the gene-altered non-human mammalian animal according to the present invention comprises a non-human mammal that can demonstrate a difference at a level larger than a detectable level in a band pattern of a DNA extracted from the gene-altered non-human mammal, when Southern blotting is performed for the DNA using a full length of the cathepsin E-associated gene as a probe, as compared with the Southern blotting for a DNA from a wild type mouse. Further, the gene-altered non-human mammalian animal may delete the cathepsin E-associated gene in a heterozygous manner or in a homozygous manner. However, the gene-altered non-human mammalian animal according to the present invention may preferably delete the cathepsin E-associated gene in a homozygous manner.

As the non-human mammalian animals, there may be mentioned, for example, a rodent including mice, rats, and so on, although they are not limited thereto.

In the following description, it is understood herein that, for brevity of explanation, the present invention will be described by taking a mouse as an example of the non-human mammalian animal and taking a mouse cathepsin E-associated gene-altered mouse as the cathepsin E-associated gene-altered non-human mammalian animal.

The cathepsin E-associated gene-altered mouse according to the present invention can be produced generally by any method as long as it can produce a gene-altered mouse that loses its function of expressing the cathepsin E-associated gene. Such a method may include, for example, a gene targeting method (e.g., Methods in Enzymology, 225:803-890, 1993). Moreover, the gene targeting method may include, for example, knockout of a gene, conditional gene targeting, and so on.

As described above, the principle of the gene targeting method is known, and this method allows a targeted gene of interest to be altered specifically by homogenous recombination on the genome of the mouse as a host, thereby resulting in alterations such as deletions of the original functions of the targeted gene.

In performing homogenous recombination, an introducing DNA is introduced into cells and replaced by a partial sequence (a targeted sequence) in the targeted gene. The introducing DNA contains an altered sequence and a homologous sequence homologous to the corresponding portion within the targeted sequence. Consequently, the introduction of the introducing DNA into the cells can perform homogenous recombination with the targeted sequence of interest originally existing in the cell and replace the targeted sequence by the introducing DNA. It is to be noted herein, however, that the targeted sequence may be a portion of the cathepsin E-associated gene or a full sequence thereof or a sequence existing in an exon or exons or intron or introns of the genomic DNA of the cathepsin E-associated gene.

In accordance with the present invention, the gene targeting method can be carried out first by isolating the genomic DNA of the cathepsin E gene to alter its functions as a sequence corresponding to the above targeted sequence. The genomic DNA to be isolated may be preferably isolated from an animal of the species identical to the animal species from which the embryonic stem cells (ES cells) are originated, because it is used for constructing the targeting vector for performing homogenous recombination of the ES cells. By isolating the genomic DNA from the animal species identical to the animal species with the ES cells originated therefrom as described herein, the homogenous recombination of the ES cells can be performed in a better and more efficient way. Further, a further better and efficient homogenous recombination of the ES cells can be realized by selecting the animal species of the same line from the identical animal species and isolating the genomic DNA therefrom.

The genomic clone of the mouse cathepsin E-associated gene can be collected by screening from a mouse genome library using the genomic DNA isolated in the manner as described above or a mouse-originated cDNA as a probe. When a cDNA probe is used, there may be used a full-length or a partial length of cDNA of the cathepsin E-associated gene.

The genomic DNA of the cathepsin E-associated gene coupled to the probe can be cleaved with a restriction enzyme and inserted into a cloning vector. As the cloning vectors, there may be generally used any plasmid conventionally used for this object. Such a plasmid may include, for example, pBluescript, pBR322, pUC and the like, although it is not limited to a particular one.

The targeting vector according to the present invention is used for homogenous recombination of the genomic DNA of the cathepsin E-associated gene in the ES cells by the cathepsin E-associated gene with the gene thereof altered in order to lose the functions thereof. Therefore, the targeting vector of the cathepsin E-associated gene according to the present invention contains a DNA fragment of the cathepsin E-associated gene with a portion altered to lose the functions thereof. The plasmid with the genomic DNA of the cathepsin E-associated gene introduced therein can be made a targeting vector by performing in vitro alterations of the genomic DNA fragment thereof.

The targeting vector according to the present invention may be produced, for example, by the method comprising cleaving the genomic DNA fragment of the cathepsin E-associated gene by the treatment with a restriction enzyme and inserting the resulting genomic DNA fragment into a predetermined position of a plasmid into which another DNA fragment having no association with the cathepsin E-associated gene, such as a selection marker gene DNA or the like, has been previously inserted, or the method comprising replacing the genomic DNA fragment of the cathepsin E-associated gene by the another DNA fragment, such as a selection marker gene DNA or the like.

As described above, the insertion of the genomic DNA fragment of the cathepsin E-associated gene or the replacement of the DNA fragment having no association with the cathepsin E-associated gene by the another DNA fragment can allow an easier screening of a homologous recombinant obtained by performing homogenous recombination with the DNA in the targeting vector using the selection marker or the like.

Further, in accordance with the present invention, it is preferred to construct the targeting vector in such a manner that the homologous recombinant can be screened in the subsequent step in an easier way. In order to realize an easier screening, it is preferred generally to produce the homologous recombinant by a two-step screening process. The two-step screening process may be composed of, for example, a first screening step in which the screening is carried out in a culture at a cell level using an agent-resistant gene introduced into the recombinant with the targeting vector and a second screening step in which the screening is carried out for the recombinant selected by the first screening step at a DNA level using PCR method, Southern blot hybridization method or the like.

As the selection marker genes, there may be used, for example, neomycin-resistant gene and hygromycin B phosphotransferase gene, etc. for positive selection, and thymidine kinase gene and diphtheria toxin A fragment gene, etc. for negative selection. These selection marker genes may be appropriately used in accordance with purposes.

As a promoter to be coupled with the above selection marker gene, there may be mentioned, for example, phosphoglyceric acid kinase-1 (PGK-1) promoter, elongation factor 2 (EF-2) promoter, MC-1 promoter, and so on. Among those promoters, it is preferred to use a promoter having a strong expression activity in the ES cells. As the promoter activity is greatly affected by a gene locus or a DNA region thereof, etc. , in which the gene targeting is to be performed, it is generally contemplated that a more active promoter is better. In this sense, it is preferred to use PGK-1 promoter.

More specifically, the targeting vector according to the present invention may be produced, for example, by cleaving a genomic DNA of the cathepsin E-associated gene with a restriction enzyme and inserting the isolated genomic DNA into a predetermined position of a plasmid.

As an alternative method, the targeting vector of the cathepsin E-associated gene according to the present invention can be produced, for example, by deleting a portion of a genomic DNA of the isolated cathepsin E-associated gene by treatment with a restriction enzyme and inserting a selection marker gene coupled with a promoter into the deleted DNA region.

In either method, the targeting vector according to the present invention has two DNA fragments of homogenous recombination regions at the predetermined positions of the plasmid in such a manner that the DNA fragment of the first homogenous recombination region is present on the upstream side and the DNA fragment of the second homogenous recombination region is present on the downstream side.

More specifically, the targeting vector according to the present invention may be constructed in such a manner that, on the one hand, the DNA fragment of the first homogenous recombination region is located on the 5'-upstream side on which a positive selection marker gene such as neomycin-resistant gene (Neo^{r} gene), etc. is situated and, on the other hand, the DNA fragment of the second homogenous recombination region is located between the 3'-downstream side end of the positive selection marker gene and the 5'-upstream side end of a negative selection marker gene such as herpes virus thymidine kinase gene (HSV-tk gene), etc.

As shown in Fig. 1, the targeting vector according to the present invention may be constructed in such a manner that two DNA fragments of homogenous recombination regions are inserted into the plasmid in the manner as described above and the DNA fragment of the first homogenous recombination region is inserted into the upstream side while the DNA fragment of the second homogenous recombination region is inserted into the downstream side. The method for the insertion of the DNA fragment into the plasmid may be any method conventionally used in the art.

Specifically, the targeting vector of the present invention can be constructed such that the DNA fragment of the first homogenous recombination region is inserted into the 5'-upstream side of the positive selection marker gene with the promoter linked thereto and the DNA fragment of the second homogenous recombination region is inserted into a region between the 3'-downstream side of the positive selection marker gene and the 5'-upstream side of the negative selection marker gene with the promoter linked thereto.

More specifically, the DNA fragment of the first homogenous recombination region inserted into the 5'-upstream side of the positive selection marker gene with the promoter linked thereto is a DNA fragment that is located on the upstream side of exon 1 of the cathepsin E-associated gene and exists in a region between a first cleavage site to be cleaved with a restriction enzyme *StuI* and a second cleavage site to be cleaved with a restriction enzyme *HindIII*. Further, it has a base sequence of approximately 1.2 kbp upstream of exon 1 and in the range from the base of base number 1,438 to the base of base number 2,656 of SEQ ID #1.

On the other hand, the DNA fragment of the second homogenous recombination region inserted into the 3'-downstream side of the positive selection marker gene with the promoter linked thereto is a DNA fragment that is located on the downstream side of exon 4 of the cathepsin E-associated gene and exists in a region between a position upstream by 76 bp from a third cleavage site to be cleaved with a restriction enzyme *ScaI* and a position downstream by 52 bp from a fourth cleavage site to be cleaved with a restriction enzyme *HpaI*. Further, it has a base sequence of approximately 7.0 kbp downstream of exon 4 and in the range from the base of base number 6,417 to the base of base number 13,548 of SEQ ID #1. Therefore, the DNA fragment of the second homogenous recombination region contains exon 5 and exon 6.

A specific example of the method for the production of the targeting vector will be described hereinafter with reference to Fig. 2.

First, the cathepsin E gene or a fragment thereof is cleaved by means of a conventional method using a restriction enzyme (for example, *HindIII*, etc.). Out of the fragments cleaved in the above manner, a DNA fragment (as indicated in Fig. 2 as Short Arm) containing a first homogenous recombination region (a *StuI-HindIII* cleavage region) is inserted into a plasmid including, for example, pUC118 by a conventional method. Then, the *StuI-HindIII* cleavage region is cleaved with a restriction enzyme, and the cleaved site is introduced conventionally into a plasmid, such as pPGKNeoE/E5 for example, into which a first selection marker gene such as Neo^{r} gene, etc. has previously been introduced, thereby yielding a pNeoHs vector. The first selection marker gene is coupled with a promoter such as, for example, PGK.

For the cathepsin E-associated gene or a fragment thereof, on the other hand, the second homogenous recombination region (containing a RV-*NotI* cleavage region as a primer) corresponding to the region located between the position upstream by 76 bp from the third cleavage site to be cleaved with the restriction enzyme *ScaI* and the position downstream by 52 bp from the fourth cleavage site to be cleaved with the restriction enzyme HpaI is amplified by the PCR method (in the drawing, referred to as Long Arm) and ligated with a vector such as pT7Blue vector, thereby resulting in the formation of a pT7-RV-*NotI* vector.

From the vector formed in the above manner, the RV-*NotI* cleavage site is cleaved with a restriction enzyme and then introduced into pNeoHs vector with the first homologous recombination region integrated therein, thereby producing a pNeoS&L vector. In this vector, the *StuI-HindIII* cleavage site as the first homologous recombination region is conjugated on the upstream side of the Neo gene and the RV-NotI cleavage site is conjugated on the downstream side of the Neo gene.

Further, the pNeoS&L vector with the *StuI-HindIII* cleavage site and the RV-*NotI* cleavage site introduced thereinto is cleaved with *SalI-NotI*, and the cleaved site is then inserted into a plasmid, such as pPGKTk-*SacII*/R, etc., with a second selection marker gene, such as thymidine kinase gene, etc., resulting in the formation of a pNeoCE vector as the targeting vector. The second selection marker gene is also conjugated with a promoter such as PGK, etc.

Next, homogenous recombination by the targeting vector will be described hereinafter.

In accordance with the present invention, the homologous recombination is performed using the targeting vector having the construction as described above to artificially recombine the altered cathepsin E-associated gene having a base sequence identical to or similar to that of the cathepsin E-associated gene on the genome of the cathepsin E-associated gene, thereby resulting in the formation of a targeted allele (refer to Fig. 1).

It is to be noted herein that a frequency of occurrences of homogenous recombination for a gene is known to be as very low as approximately 10⁻⁶. In the case, therefore, where the genetically homogenous recombination is performed using an oosperm in order to obtain the homologous recombinant of interest, it is theoretically needed to perform genetically engineered recombination operations for more than 10⁶ oosperms and to screen at least several tens to several hundreds of recombinant samples. It is practically impossible, however, to use such a large number of oosperms for genetically engineered recombination operations so that a pluripotent cell is actually used which has a pluripotency like an oosperm and which is capable of being cultured in vitro. As such pluripotent cells, for example, ES cells originating from a mouse are generally used and may include, for example, mouse-derived embryonic stem cells (ES cells), embryonic cancer cells (EC cells), and so on. The ES cells may further include, for example, TT2 cells, AB-1 cells, J1 cells, R1 cells, E141 cells, and so on.

Then, the present invention will be described by taking the mouse-derived embryonic stem cells (ES cells) as an example, although it is not limited to the cells.

The targeting vector integrated with the DNA fragment having no function of the cathepsin E-associated gene in the above manner is then introduced into the ES cells. As the method for the introduction of the targeting vector into the ES cells, there may be used, for example, electroporation method, microinjection method, calcium phosphate method, DEAE-dextran method or the like, although the electroporation method is preferred. The introduction of the targeting vector into the ES cells by the introduction method can cause homogenous recombination of the genomic DNA fragment (a targeted sequence) of the cathepsin E-associated gene with the DNA fragment of the cathepsin E-associated gene (an altered sequence of interest) with its functions lost to occur in the ES cells, resulting in a substitution of the altered sequence for the targeted sequence of interest.

Then, the cells are selected which underwent homogenous recombination of the targeted sequence of the cathepsin E-associated gene with the altered sequence in the targeting vector in the manner as described above.

The selection of the homologously recombined cells may preferably be conducted in the second screening step in order to investigate whether the cathepsin E-associated gene of interest is certainly targeted. The selection of the genetically recombined cells can be carried out, for example, by removing non-recombined cells having no Neo^{r} gene by the addition of G418 to a cell culture and removing the remainder of cells randomly recombined with a HSV-tk gene by the addition of gancyclovir. The cathepsin E-associated gene of the genetically recombined cells selected is a variant sequence with the Neo^{r} gene inserted in its coding sequence and cannot produce cathepsin E.

The ES cells which underwent the genetically homogenous recombination in the above manner are then injected into an embryo of the 8-cell stage or an early embryo of a mouse blastocyst. As the method for injecting the ES cells into the embryo, there may be used, for example, micromanipulation method, agglutination method, and so on, although it is not limited to a particular one, and any method can be used as long as it can achieve the objects of the present invention. The early embryo injected with the ES cells and underwent homogenous recombination is then transferred into the uterus of a female mouse as a pseudo-pregnant foster mother, resulting in giving birth of chimeric mice.

When a mouse is used, a female mouse is subjected to hyperovulation treatment with a hormone agent such as, for example, PMSG having a FSH-like activity and hCG having an LH action and mated with a male mouse. In a given period after offspring, early embryos are collected from the uterus of the mouse and then injected,in vitro with the homogously recombined ES cells using the targeting vector, resulting in the formation of chimeric embryos.

The pseudo-pregnant female mouse to be used for a foster mother may be produced by mating a female mouse of a normal reproduction cycle with a castrated male mouse. To the resulting pseudo-pregnant female mouse is then implanted the chimeric embryo produced in the above way, and a chimeric mouse is produced by offspring and giving birth. In order to become implanted with the chimeric embryo and become pregnant for sure, it is preferred that the pseudo-pregnant mouse as the foster mother and the female mouse from which a fertilized egg is recovered are selected from the identical female mice group having the equal reproduction cycle.

The chimeric mouse originated from the ES cell-implanted embryo is then selected from the chimeric mice produced in the manner as described above. After the selected chimeric mouse originated from the ES cell-implanted embryo has matured, the mouse is mated with a male mouse of another appropriate line, such as, for example, a male mouse of a pure line, resulting in giving birth of baby mice.

In the case where a germ cell of the chimeric mouse is originated from the above homologous recombinant, that is, the cell with the cathepsin E-associated gene deleted therefrom, the mice of the next generation can be produced as heterozygous variant mice in which one of the cathepsin E-associated genes is an altered heterozygote. Mating the heterozygous variant mice having such a heterozygote (+/-) with each other can produce transgenic mice as homozygous variant mice having a homozygote (-/-) with no normal cathepsin E.

In order to confirm the introduction of the ES cells into the germ line of chimeric mice upon giving birth of mice of the next generation, it is possible to confirm the introduction of the ES cells into the germ line on the basis of various characters as indicators. Among various characters, it is easy to identify fur colors of mice of the next generation for confirmation of the introduction of the recombinant ES cells because the mice of the next generation are bred with original fur colors derived from the recombinant ES cells. The mouse with the recombined ES cells implanted into the embryo introduced into the germ line is selected in the manner as described above, and the chimeric mouse is bred to produce an individual with the gene of interest deleted therefrom. Further, the resulting heterozygous mice with the cathepsin E-associated gene deleted therefrom are mated with each other, resulting in the production of the homozygous mouse with the gene of interest deleted therefrom.

The present invention will be described hereinafter in more details by way of examples. It is to be understood, however, that the-present invention is not interpreted in any respect as being limited by or to the following examples and the following examples are illustrated solely with the intent to allow the present invention to be understood more clearly.

### Examples

### Example 1: Production of mouse with deleted cathepsin E gene

Cathepsin E gene was cloned in a 129/Sv mouse library, and a sequence located of approximately 1.2 kbp upstream from exon 1 for a DNA fragment for the first homologous recombination region and a sequence of approximately 7.0 kbp downstream from exon 4 for a DNA fragment for the second homologous recombination region were inserted into a plasmid integrated with neomycin-resistant gene. The genome of the DNA fragment for the first homologous recombination region of approximately 1.2 kbp and the DNA fragment for the second homologous recombination region of approximately 7.0 kbp was designed to assume a form bridging the neomycin-resistant gene, and thymidine kinase gene was inserted into the position downstream of the genome of approximately 7.0 kbp.

The resultant plasmid was then inserted into an ES cell by electroporation to cause an occurrence of homologous recombination. The resultant homologous recombinant was subjected to double selection using G418 and GANC (gancyclovir), resulting in the production of the ES cell with the cathepsin E gene of interest deleted therefrom. More specifically, this selection was conducted in order to remove non-recombined cells having no Neo^{r} gene by the addition of G418 to a cell culture and further remove randomly recombined cells with HSV-tk gene left therein by the addition of GANC (gancyclovir). The cathepsin E gene of the genetically recombined cell selected was a variant sequence with the Neo^{r} gene inserted into the coding sequence and cannot produce cathepsin E.

The resultant ES cell with the cathepsin E gene deleted was injected into the blastocyst collected from CS57BL/6 mouse by microinjection method and then implanted on the uterus of a mouse foster mother.

Next, a newborn chimeric male mouse was mated with a CS57BL/6 female mouse, resulting in giving birth of heterozygous mice. A heterozygous type of the cathepsin E gene was confirmed by extracting DNA from the tail of a newborn mouse.

Finally, the male and female mice, each of a heterozygous type, were mated with each other and bred mice (of a homozygous type) with the cathepsin E gene deleted completely.

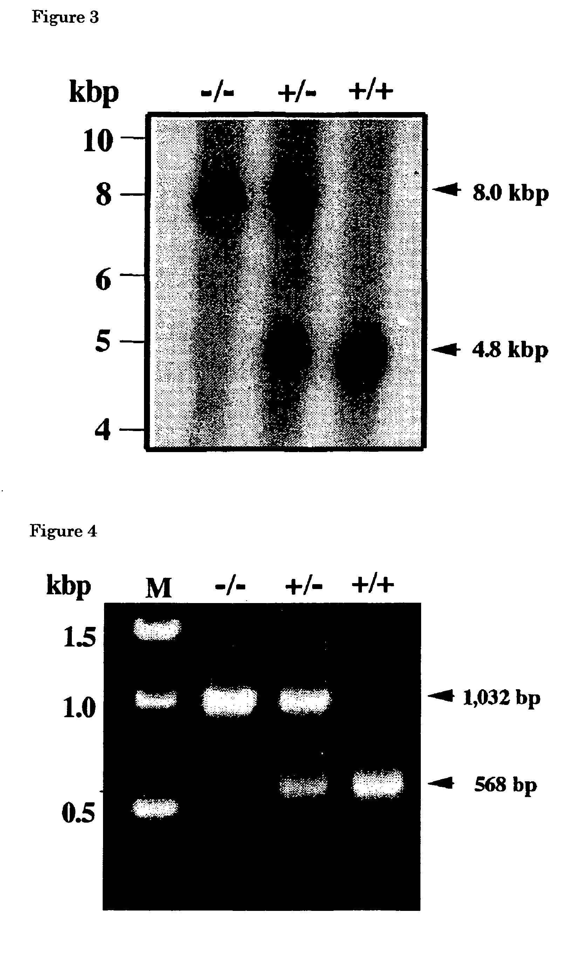

Whether the resultant mouse had a heterozygote (+/-) or a homozygote (-/-) was confirmed by subjecting the DNA extracted from the tail of the mouse to Southern blot analysis and PCR method. Before confirmation, the extracted DNA was digested with *KpnI* and hybridized with a probe as shown in Fig. 1. The results of the Southern blot analysis are shown in Fig. 3, and the results of the PCR method are shown in Fig. 4. In the drawings, symbol (+/+) refers to a wild-type mouse, and reference letter M in Fig. 4 stands for a marker.

### Example 2: Construction of targeting vector

The above targeting vector was produced in the manner as shown in Fig. 2. A short arm as a fragment containing the DNA fragment for the first homologous recombination region is a DNA fragment composed of a *StuI* (5'-side) - *HindIII* (3'-side) cleavage site obtained by cleaving the cathepsin E gene with restriction enzymes *StuI* and *HindIII*, respectively.

Separately, a long arm (a RV-*NotI* fragment) as a fragment for the second homologous recombination region was obtained by amplifying a DNA fragment of the cathepsin E gene by means of T-taq PCR, the DNA fragment being located in a region between the region upstream by 76 bp from a cleavage site (third cleavage site) to be cleaved with a restriction enzyme *ScaI* and the region downstream by 52 bp from a cleavage site (fourth cleavage site) to be cleaved with a restriction enzyme *HpaI*.

The resulting *HindIII-HindIII* cleavage site was then inserted into the plasmid pUC118, and the plasmid pUC118 was digested with *StuI*, following by linking a *HindIII* linker to the 5'-terminal side thereof and digesting with *HindIII*, resulting in the production of a *HindIII-HindIII* fragment (3'-side) as the short arm. This fragment was then inserted into plasmid pPGKNeoE/E5, resulting in the formation of a vector pNeoHs with the short arm introduced into the *HindIII-HindIII* cleavage site on the 5'-upstream side of the Neo gene.

The RV-*NotI* fragment as the amplified long arm was ligated with a vector pT7Blue, resulting in the formation of a vector pT7-RV-*NotI* which in turn was digested with RV-*NotI* to obtain the RV-*NotI* fragment as the long arm.

On the other hand, the vector pNeoHs was digested with *SmaI-NotI*, and the cleaved RV-*NotI* fragment was linked to the 3'-downstream side of the Neo gene, resulting in the formation of a vector pNeoS&L. The linkage of the cleaved RV-*NotI* fragment with the Neo gene was carried out by linking the *SmaI* cleavage site of the pNeoHs vector to the RV cleavage site of the Neo gene.

In order to introduce thymidine kinase gene as another selection marker gene, that is, a negative selection marker gene, there was produced a PGK-conjugated plasmid with thymidine kinase gene (TK) introduced therein, pPGKTK-*SacII*/R. This pPGKTK-*SacII*/R plasmid was then digested with *NotI* and *SalI*.

At the same time, the pNeoS&L vector was digested with *NotI* and *SalI*. This digestion allows the *NotI* cleavage site of the RV-*NotI* fragment of the pNeoS&L vector to be linked to the thymidine kinase gene (TK), resulting in the formation of a vector pNeoCE as a targeting vector.

### Example 3: Analysis of hemocytes

Blood was collected from the cathepsin E gene-deleted mice produced in Example 1, and erythrocytes, leucocytes and platelets were separated as hemocytes from the collected blood to carry out an analysis for hemocytes under clean and aseptic environments. As control mice, there were used C57BL/6 mice of wild type. The results of the analysis for the number of hemocytes under clean environment are shown in Table 1 below. The results of the analysis for the number of hemocytes under aseptic environment are shown in Table 2 below. It is to be noted herein that the hemocytic analysis revealed no significant difference between the deleted and control mice. The analysis for the hemocytes was carried out by ELISA method. In the examples which follow, the analysis was conducted in the same manner.

**Table 1:**

| Analysis for hemocytes [under standard laboratory (conventional) environment] | | | |
|---|---|---|---|
| | No. of Erythrocytes (x 10⁴/µl) | No. of Leucocytes (x 100/µl) | No. of Platelets (x 10⁴/µl) |
| Control Mice (n=5) | 752 ± 104 | 27.5 ± 13.8 | 78.2 ± 14.8 |
| Deleted Mice (n=11) | 802 ± 198 | 30.5 ± 8.4 | 74.8 ± 25.3 |

**Table 2:**

| Analysis for hemocytes (under aseptic environment) | | | |
|---|---|---|---|
| | No. of Erythrocytes (x 10⁴/µl) | No. of Leucocytes (x 100/µl) | No. of Platelets (x 10⁴/µl) |
| Control Mice (n=4) | 824 ± 69 | 32.8 ± 12.7 | 72.8 ± 21.1 |
| Deleted Mice (n=5) | 810 ± 79 | 35.5 ± 17.3 | 77.1 ± 12.8 |

### Example 4: Analysis for number of leucocytes classified by kinds

The leucocytes of the blood samples collected from the cathepsin E gene-deleted mice produced in Example 1 and the control mice were further divided into neutrophils, lymphocytes, monocytes, eosinophils, and basophils and analyzed for their numbers. The results of the analysis for the number of leucocytes by kinds under standard laboratory (conventional) environment are shown in Table 3 below. The results of the analysis for the number of leucocytes by kinds under aseptic environment are shown in Table 4 below. It was found from the analysis results that the number of eosinophils under standard laboratory (conventional) environment as shown in Table 3 was statistically significant at a 1% significance level and the number of monocytes under aseptic environment as shown in Table 4 was statistically significant at a 5% significance level. These results reflect the characters of symptoms of an atopic disease.

**Table 3:**

| Number of leucocytes by kinds (under conventional environment) | | | | | |
|---|---|---|---|---|---|
| | No. of neutrophils (x 100/µl) | No. of lymphocytes (x 100/µl) | No. of monocytes (x100/µl) | No. of eosinophils (x 100/µl) | No. of basophils (x100/µl) |
| Control Mice (n=5) | 3.8 ± 2.1 | 18.1 ± 14.1 | 2.7 ± 1.4 | 0.3 ± 0.2^{a} | 0 |
| Deleted Mice (n=11) | 5.6 ± 2.2 | 20.7 ± 6.7 | 4.1 ± 2.5^{b} | 1.2 ± 0.6^{a} | 0 |

| | | | | | |
|---|---|---|---|---|---|
| a: statistically significant at a 1% significance level | | | | | |

**Table 4:**

| Number of leucocytes by kinds (under aseptic environment) | | | | | |
|---|---|---|---|---|---|
| | No. of neutrophils (x 100/µl) | No. of lymphocytes (x 100/µl) | No. of monocytes (x 100/µl) | No. of eosinophils (x 100/µl) | No. of basophils (x 100/µl) |
| Control Mice (n=4) | 2.3 ± 0.9 | 28.1 ± 10.8 | 1.98 ± 0.9 | 0.4 ± 0.3 | 0 |
| Deleted Mice (n=5) | 4.0 ± 2.2 | 30.1 ± 15.1 | 1.66 ± 1.54^{b} | 0.6 ± 0.7 | 0 |

| | | | | | |
|---|---|---|---|---|---|
| b: statistically significant at a 5% significance level | | | | | |

### Example 5: Analysis for properties of erythrocytes

In order to analyze the properties of erythrocytes of the blood samples collected from the cathepsin E gene-deleted mice produced in Example 1 and the control mice, measurements were conducted for an amount of hemoglobin, a hematocrit value, an average volume of erythrocytes, an average amount of erythrocytic hemoglobin, and an average concentration of erythrocytic hemoglobin under clean environment. The results are shown in Table 5 below. No significant difference was recognized in the analysis for the properties of the erythrocytes.

**Table 5:**

| Analysis for properties of erythrocytes (under conventional environment) | | | | | |
|---|---|---|---|---|---|
| | Amount of hemoglobin (g/dl) | Hematocrit value (%) | Average erythrocyte volume (fl) | Average erythrocyte hemoglobin amount (pg) | Average erythrocyte hemoglobin conc. (g/dl) |
| Control Mice (n=5) | 12.3 ± 1.5 | 43.3 ± 5.6 | 56.4 ± 1.5 | 16.1 ± 0.7 | 28.4 ± 0.5 |
| Deleted Mice (n=11) | 11.8 ± 2.0 | 41.7 ± 6.3 | 54.8 ± 1.0 | 15.5 ± 0.5 | 27.6 ± 1.1 |

### Example 6: Serological analysis

The cathepsin E gene-deleted mice produced in Example 1 above and the control mice were subjected to a serological analysis under clean environment. The serological analysis was carried out for a total protein mass, GOT (asparagic aminotransferase to be utilized for the diagnosis of liver diseases and myocardial infarction), GPT (alanine aminotransferase to be freed at the time of a cardiac disease), an amount of blood uric nitrogen, and creatinine. The results for the serological analysis were shown in Table 6 below. No significant difference was recognized in the serological analysis.

**Table 6:**

| Serological analysis (under conventional environment) | | | | | |
|---|---|---|---|---|---|
| | Total protein mass (g/dl) | GOT (IU/l) | GPT (IU/l) | Blood uric nitrogen amount (mg/dl) | Creatinine (mg/dl) |
| Control Mice (n=5) | 4.8 ± 0.303 | 38.4 ± 14.45 | 19 ± 6.44 | 18.24 ± 4.44 | 0.32 ± 0.04 |
| Deleted Mice (n=9) | 4.6 ± 0.366 | 35.0 ± 4.24 | 20 ± 4.98 | 17.71 ± 4.44 | 0.3 ± 0.09 |
| GOT: asparagic aminotransferase to be utilized for the diagnosis of liver diseases and myocardial infarction GPT: alanine aminotransferase to be freed at the time of a cardiac disease | | | | | |

### Example 7: Amount of antibodies classified by kinds

The cathepsin E-deleted mice produced in Example 1 above and the control mice, both grown under standard laboratory (conventional) environment, were measured for amounts of antibodies classified by kinds. The antibodies measured were IgG1, IgG2, IgM, and IgE. The results are shown in Table 7 below. The results of Table 7 revealed recognition of a statistical significance for IgE at a 1% significance level. The results greatly reflect characters of symptoms of atopic diseases.

**Table 7:**

| Amounts of antibodies by kinds (under conventional environment) | | | | |
|---|---|---|---|---|
| | IgG1 (µg/ml) | IgG2 (µg/ml) | IgM (µg/ml) | IgE (ng/ml) |
| Control Mice (n=7) | 1714 ± 476 | 47.7 ± 6.3 | 621.6 ± 107.1 | 98.1 ± 41.4^{a} |
| Deleted Mice (n=8) | 1922 ± 369 | 49.3 ± 11.8 | 608.2 ± 191.8 | 1051.2 ± 400^{a} |

| | | | | |
|---|---|---|---|---|
| a: statistically significant at a 1% significance level | | | | |

### Example 8: Amounts of cytokines secreted from splenocytes

The cathepsin E-deleted mice produced in Example 1 above and the control mice, grown under standard laboratory (conventional) environment, were measured for amounts of cytokines secreted from their splenocytes. The cytokines measured were IL-4, IL-5, IFN-gamma and IL-2. The results are shown in Table 8 below. It was found from the results of Table 8 that a statistical significance was recognized for IL-4 and IL-5 at a 1% significant level. These results suggest that the differentiation into Th-2 cells out of two kinds of effecter T cells was accelerated to a considerable extent for the cathepsin E-deleted mice, resulting in an occurrence of allergy by humoral immune response.

**Table 8:**

| Amounts of cytokines secreted from splenocytes (under conventional environment) | | | | |
|---|---|---|---|---|
| | IL-4 (pg/ml) | IL-5 (pg/ml) | IFN-γ (pg/ml) | IL-2 (pg/ml) |
| Control mice (n=8) | 162 ± 20^{a} | 78 ± 38^{b} | 3010 ± 2460 | 6345 ± 2380 |
| Deleted mice (n=8) | 952 ± 526^{a} | 224 ± 58^{b} | 2820 ± 2650 | 6771 ± 2100 |

| | | | | |
|---|---|---|---|---|
| a, b: statistically significant at a 1% significance level | | | | |

### Example 9: Experiments for induction of experimental contact dermatitis by pasting with hapten

The cathepsin E-deleted mice produced in Example 1 above, the control mice, and NC/Nga mice were subjected to experiments for induction of experimental contact dermatitis by pasting with hapten.

The experiments were carried out by preparing a 5% solution of hapten (picryl chloride) in a mixture of ethanol with acetone at the rate of 4 to 1 (ethanol : acetone). From one week after immunological induction, the 5% solution was locally pasted on the back of the experimental mice in the amount of 150 µl once at intervals of three or four days and symptoms of atopic dermatitis were observed.

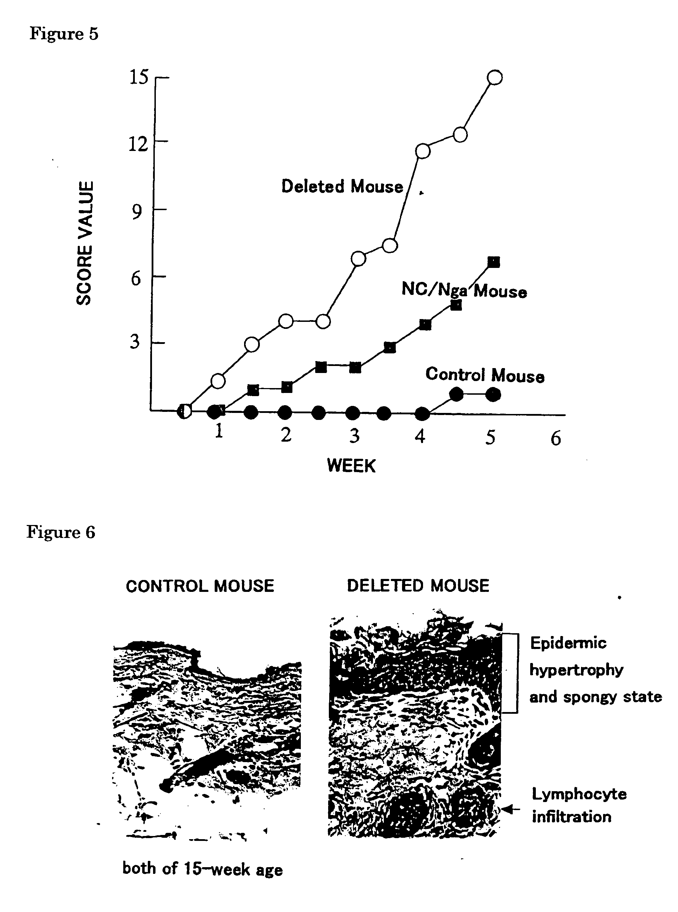

During the period of experiments, the following five items were observed and they were assessed by four-score ratings for each item and compared by total scores for all the items. The items observed include: (1) ruber or bleeding; (2) edema; (3) falling-off of hair or tissue defect; (4) skin drying; and (5) rash. Each item was assessed by the following score ratings: score 0 (not observed); score 1 (light) ; score 2 (medium) ; and score 3 (severe) . The results are shown in Fig. 5.

From the results as shown in Fig. 5, it is suggested that the cathepsin E-deleted mouse according to the present invention can be used as a standard experimental animal model for experiments inducing contact dermatitis.

Fig. 6 shows the results for the histopathological analysis for the skin of each of the control mice and 15-week aged mice with the cathepsin E deleted. The results of the histopathological analysis reveal that the hypertropy and a spongy state of the epidermis were recognized to a considerable extent and lymphocytes were infiltrated for the cathepsin E-deleted mice according to the present invention. On the other hand, no histopathological changes by the atopic dermatitis were recognized for the control mice.

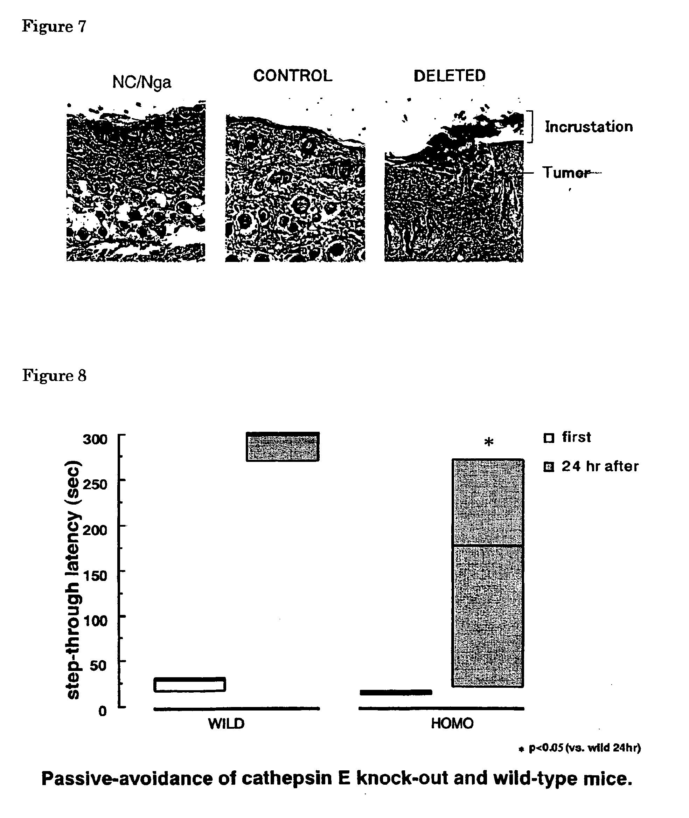

Fig. 7 shows the pathological results for the skin by contact dermatitis in the experimental mice when hapten was pasted for five months in the above experiments. Fig. 7 reveals a partial infiltration of lymphocytes in the control mice. Further, as compared with the control mice, NC/Nga mice demonstrated a considerable extent of lymphocytic infiltration and hypertropy of the epidermis. On the other hand, the formation of abscess by lymphocytes and incrustation was recognized in the cathepsin E-deleted mice according to the present invention.

### Example 10: Experiments on learning disabilities and memory impairments

The cathepsin E-deleted mice according to the present invention were subjected to experiments using a passive avoidance device of a step-through trial-and-error type (composed of two compartments (light and dark chambers) and a floor with a stainless grid disposed so as for electrical stimulation to be applied from the floor in the dark chamber) . As a result, it could be judged that the cathepsin E-deleted mice caused an occurrence of learning disabilities and memory impairments.

Fig. 8 shows the results of measurements for the cathepsin E-deleted mice and the control mice used as experimental animals. The experiments were conducted by placing the mouse in the light chamber and, when the mouse moved into the dark room, allowing electric stimulation to be applied from the floor (at an electric stimulation interval of 1 second for a period of 10 seconds of electric stimulation). As a learning step, the time for transfer from the light chamber to the dark chamber was measured as a step-through latency. After 24 hours, the mouse was placed again in the light chamber and the step-through latency was then measured. It was apparent from the results that the step-through latency was shorter for the cathepsin E-deleted mouse according to the present invention than for the control mouse so that learning disabilities or memory impairments were caused to occur for the cathepsin E-deleted mouse. Further, as no difference in autokinesis was recognized between the deleted mouse and the control mouse, these results suggested unique symptoms similar to human Alzheimer's disease or symptoms of mental depression.

### Example 11: Experiments on the acceleration of fighting episodes

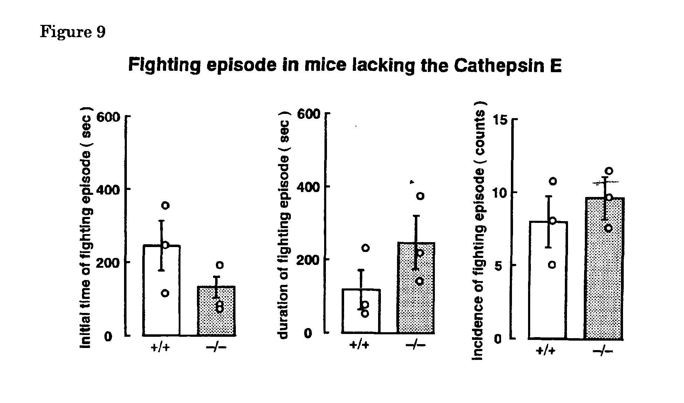

A single mouse with the cathepsin E-deleted and a single control mouse were placed each in a single cage and bred independently for 30 days or more. Then, the two mice were placed together in a single cage in order to observe expression of their fighting episodes during a period of 10 minutes. As items for the fighting episodes, actions including a conflicting action by rising, a biting action, crying action, etc. were measured as a start time (A) of such fighting episodes, a duration time (B) and frequency of occurrences of actions (C) . As was apparent from Fig. 9, although no statistic significance was recognized between the two mice for the frequency of the fighting episodes, the cathepsin E-deleted mouse was found to be shorter in the start time of fighting episodes and longer in the duration time than the control mouse. These symptoms suggested a similarity to human schizophrenia-like symptoms or symptoms of human mental depression.

### Example 12: Histological analysis for states of the gastric mucosa

A normal mouse and a cathepsin E-deleted mouse, each 24 weeks of age, were placed in a water vessel in order to apply stress for several minutes, and the states of the gastric mucosa of each mouse were subjected to the histological analysis by microscopy. It was found from the results of the histological analysis that the cathepsin E-deleted mouse exhibited a remarkable extent of infiltration of inflammatory tissues such as neutrophils, lymphocytes and so on and even bleeding.

### Example 12: Experiments of frequency of occurrences of ulcer by a hydrochloric acid load test

A normal mouse and a cathepsin E-deleted mouse, each 24 weeks of age, were subjected to a hydrochloric acid load test for a comparison of a frequency of occurrences of the ulcer. The results revealed that, obviously, the ulcer was more likely to occur for the cathepsin E-deleted mouse than the normal mouse and that there was shown the tendency of becoming more severe.

### Industrial Applicability

The non-human mammalian animals with the cathepsin E-associated gene altered according to the present invention can be utilized as an experimental animal model which are remarkably useful for studies on the clarification of various disease conditions in which the cathepsin E-associated gene is contemplated to be involved, and for the development of methods for the therapy of such diseases as well as which has a definite genetic background.

The non-human mammalian animals with the cathepsin E-associated gene altered according to the present invention can also be utilized for the elucidation of allergic diseases such as atopic dermatitis and so on and the disease conditions thereof. Further, the cathepsin E-associated gene-altered non-human mammalian animals according to the present invention are expected to be utilized as an experimental animal model for use in experiments for clarification of learning disabilities and memory impairments as well as acceleration of fighting episodes, so that they are expected to contribute greatly to making such functions clear. Moreover, the non-human mammalian animals with the cathepsin E-associated gene altered according to the present invention are expected to assist in making clear the physiological functions of the cathepsin E against stress because they have a high sensitiveness to stress.

Furthermore, the cathepsin E gene according to the present invention is useful for the production of the cathepsin E-associated gene-altered non-human mammalian animals, such as cathepsin E gene-deleted mice and so on. Eventually, the produced cathepsin E gene-altered non-human mammalian animals can greatly contribute as an experimental animal model to studies on diseases and conditions of diseases in which the cathepsin E gene is involved.

In addition, the targeting vector according to the present invention is useful for the production of the cathepsin E-associated gene-altered non-human mammalian animals, in which the expression of the cathepsin E-associated gene is suppressed by performing homologous recombination through the homologously recombined DNA fragment and the functions of the cathepsin E gene are made inactive. Therefore, it can greatly contribute to studies on diseases and conditions of diseases in which the cathepsin E genes are involved.

SEQUENCE LISTING

<100> Kyushu TLO Company, Limited

<120> CATHEPSIN-ASSOCIATED GENE-ALTERED NON-HUMAN MAMMALIAN ANIMALS

<130> MP101336-EP

<140> EP 03723398

<141> 2003-05-26

<150> JP P2002-153641

<151> 2002-05-28

<160> 1

<210> 1

<211> 18352

<212> DNA

<213> Mus musculus

<400> 1

ggtaccttgg gtatgcagtg agaccatgtc tcaaagaaca agcacatctt tatgaacgtg 60

aatgagaaca tttgttacca caccaggccc tgtacatacc ttttattctt ccaagcttgc 120

aaaacatctg ccatggcaga aattaatctc tccctctttc tttagcttct ctatctccct 180

ctttcagaaa ggggttctag aacataagac cctctagaag agtggttctc aacctcccta 240

atgttgggac cccttaatat agtttcttgt gttgtggtga cccccaacca taaaattctt 300

tttcattatc atttcataat tgtaattttc ctgctgttag aaatcatggt gttatgaatt 360

acaacgtaaa tatctgatat gcagatgtat ggctcctaaa ggggttaaga acccctactc 420

tggatccttt agtacagtgc catggttagg aacatcagct ttaaagttag gccaacctgg 480

gtttggaatg gggttacagc actggcacgg gagccatcct gggtctccat ttcctcacgt 540

gtgaaacggc tttaaatatc ctcgaaggat tggcatgagg tctgaagaaa tacttctaag 600

tcctttaaca tagcatctga gacacagcta cgccctcccc actgtctctt gttctcatta 660

ttcaatttaa cctcttcatt ttagacatga agacatgaag ttctgaaaga aattgcttta 720

tggccaggtg gtcagttagg gcaaaactag gtggatccaa gcatcctggg ccagtctctc 780

tatgccttgc cacctactgc acagtggatt ttcctttatc cctcggctct gagcagaaag 840

aggttaggac attttcttaa ctccttgtca gggtcatttt gagctgatgc tcttgccaga 900

gatggagaca ggggctcctg gagtgtagct ccctgccata aggagccagc atgtatccca 960

caccagacca gctgactccc atatagcact gtgcagctgc ccctgtcatt attttatcct 1020

tgtcaatctg aaactataaa aagagaagct aattaggtaa tgtcacgtgt caccaccaac 1080

agcttcccca cattctggga actgtcaaac ctctgcatgc agctagccat tcctggagag 1140

ccctcttgtc gaggctcctt tgcacgtgct gagactggat ggctgcctac attgtgtcct 1200

ttgccccatg tgccaatttc taatgttctc tatgattttt cccagaggtc tcacacacac 1260

atagaccctc aaatgcttag ggcttttgga tccagaaatg gctcgcacca tccttaacaa 1320

tggtccacag tagttttccg tttcagtctg ggaggatcca gtgagccagg ttaggtgagg 1380

ttcctggtgt ttccagaaag gacagagccc atcagctctg ataaaagtcc ctacaggcct 1440

gctatcaatc tttctcagac tttcccaagc tcagcagacc ctattaacag acaggcagac 1500

tgagtcttcc tcctcaaggg tcaagtaact catagttgcc caggtctcat ttaaacatgg 1560

tctaacctaa ccctacacat ttaataaaca gatggggcaa ggtcagagat ggagaaggag 1620

agggcttagg tatgctggca tcaatagctc agtgatgtat gcagaaagag ccctggacta 1680

gcagttaaga agcctgggtc accctagccc agccctaatg acctgtgcca ttggagatca 1740

cttgagctct cgctgaccat gttcccatct gctgtgcaag tggattggtc ttgaggacct 1800

ggaggcactc ttccagccca aggttttctc agtgaccgta tcctgaccat gggatgtgtt 1860

aaagtgtttt cagtgtgtcc acattccttg ttttttgcac ttaaccttcc taaaaaccca 1920

gatgctgttg ctgaggtgtc tggtcaggca actgtgagca accatgacag agcttggttc 1980

tggacaagtg acgagccccc ctccagcgtg ctgttcttca gggagcagac aggcccctca 2040

cccttgtggg cagtcagagg atgggctccc aggaaacgta aatgctagag aggtgtggtg 2100

gctttgggga caaggaaagt cagttttgca tttccctcct tttttgggtg tttcttcaat 2160

aatttggatc tcacaacagg ttcggcctgg cttcagaccc aattttggct tctctggcat 2220

cagagataaa aggcaaggtg caggcacttt gccataacac taactggcac agacagcgat 2280

aaccctaacc caggcacccc attattttat taccagagct ccctgggggg actggtgttc 2340

ccatcccagc tcagaagcca ttgactaaga agatggggca agaaggtttt ccgtcctgtc 2400

acccctacct ctcttaagtc aggaaagttg aagaaggaaa atcacagggt ggggttgatg 2460

gtaagaaaac ttgactttcc aacctcywsg atcatgagac agtagatgct gcttaagcca 2520

ytgacttgtg gtacattgtt atggcagccc tggtacacca acacagactt gctccagtct 2580

cggctcctcc cttgcctcct cactcccttc catacaaacc ccagagccag atccgtcagg 2640

tttccagaat caggaaagct tgccccgcaa atcaatagcc agactgtccc actccaggac 2700

ctgtggtgag caagcactgg tttgctctga gtaagacaag ttgctttgag agcctctgga 2760

ggaatgcagg gagaatatga aatatgagaa caaaagcctg gctaatgttt tgagccacaa 2820

tcatgctaca cttatatcat tttggcttct gcccttctcg agtcttcaac tgaacattca 2880

tagtagcagt tcaacaaggt gggaccctca ttcacttttg cacacctaga caataaacat 2940

ggggtttctt acagtcaatg ttgcatgccc aaagaacttg aaagaagctg agacctcaac 3000

accctcattt tcagagagtg tatacacaca cacacacaca cacacacaca cacacacaca 3060

cacacacaca gaggttttgc ccttagctcc ccaagcataa gaaaggaccc caggggcaga 3120

caagggacgt cctattacac atagtgttta ctctgggcat ggctattgac tccagacctt 3180

atcattgggt cctcagactg ggcagggcag gtctgggcac caggaagagc cagtgcccct 3240

gccctgtgat caagaggaaa gaagggtgga agggagatcg gagcagagtg agagagaagc 3300

taccccttgg accaca atg aag ccc ctc ctt gtg ctg ctg ctg ctg ctg ctc 3352

Met Lys Pro Leu Leu Val Leu Leu Leu Leu Leu Leu

1               5                   10

ctg gat ctg gct cag gcc caa ggt gct ctg cac agg ta aggccctgtc 3400

Leu Asp Leu Ala Gln Ala Gln Gly Ala Leu His Arg

15 20 24

tccagcacta agttcaattg aggcatcccc caaccccaac ccagggctgc cccgggggag 3460

gaaagctaat gaggaaatga aaagaagaaa ccgggatctc tcgggaatcc ctagagaaga 3520

ttccccaggc ctctccttcc agtgtaggtg cctgcctcag ctcaagggac tccccctctg 3580

ttgttcaaag gtgtgctcca tagatgtgat ttgtgactcc aagccttcaa atactttccc 3640

aagggggcct gagcactgct atccacaggg tccttccatc cctttaagtc agccctttat 3700

gatcagacta tagactgtgt ctgacctccc attccctaga aaatctcaaa aggccagtgc 3760

ccaggaggga aagccatttc tacccaaatt ctgagtctgc aagacaaagc cattgctgct 3820

tctggtcttg aaggttccct cctgggtctt gctgtagtca ggctctgaac aggtatagag 3880

gcaatgggag cctttattta taaatgcccc tttatttcct gatctttctc caga gtg ccc 3940

Val Pro

1

ctc aga aga cat cag tcc ctt cgg aag aaa cta cgg gcc caa gga cag 3988

Leu Arg Arg His Gln Ser Leu Arg Lys Lys Leu Arg Ala Gln Gly Gln

5 10 15

ctc tca gaa ttc tgg agg tct cat aac ttg gac atg acc cga ctc agc 4036

Leu Ser Glu Phe Trp Arg Ser His Asn Leu Asp Met Thr Arg Leu Ser

20 25 30

gag tcc tgt aat gtg tat tcg agt gtc aat gaa ccc ctc atc aac tac 4084

Glu Ser Cys Asn Val Tyr Ser Ser Val Asn Glu Pro Leu Ile Asn Tyr

35 40 45 50

ctg gat gtaagatttc tcaaattgaa gcctgaggac actgggtgta gggaggaagg 4140

Leu Asp

52

ctggggcctt cagcaggggc cagtgctgca ttggtggcat tatgatcagg catggatttg 4200

tttgcttggg ggattcagac agtcccccag tctctatcta ctctgtaaag caggcttcta 4260

aatgatccag tatgcagagc tttcttcaag aggaaagaga taagtatgat atgtgttcca 4320

aaaagaaaga cccctactgt gtgcccagct gggcatttta ctagctattc ctctgggcaa 4380

agaacttggt ttgccacacc tccaggtccc tgatgacatc gtgaggatca ggataatgag 4440

agaaggatgg acccaagttt cctgggctat cctcagtttc ctccctggtg gtccttgctc 4500

tgttccttcc atcctttgcc tccagccttg cctcttctgg gctctgtaga ttgtagcttg 4560

tggtacttca tgatttagtg cccttcccct ccactccacc cacacactct ctctctctct 4620

ctcttcccta cag atg gaa tac ttt ggc acc atc tcc atc ggc acc ccg 4672

Met Glu Tyr Phe Gly Thr Ile Ser Ile Gly Thr Pro

1 5 10

ccg cag aac ttc act gtc atc ttt gac acc ggt tca tcc aac ctc tgg 4620

Pro Gln Asn Phe Thr Val Ile Phe Asp Thr Gly Ser Ser Asn Leu Trp

15 20 25

gtc cct tct gtg tac tgc acc agc cca gca tgc agtaagtgaa cttggcagtg 4770

Val Pro Ser Val Tyr Cys Thr Ser Pro Ala Cys

30 35 39

gcgggagctc actaggctaa gatggtacct tgtggcatag agagactcag gttctctcca 4830

cttatcttcc ctgccatact ggcttcccct tcctatatct gtttgttgtg cctgagccaa 4890

gcacagacaa gaaaggcaac taccagttaa gataacaacc ttcatgttca ttagaattga 4950

tggagagaaa attagccatt aggacgtggt gactcacttc ctttggaaca cccaaagcaa 5010

catgggggaa agttccttcg gtctgtacct gaaccgatag ctagattgaa ttctaaggaa 5070

aactgtgctc ctgctccagg gaggccctga tgcactggct cccttggagg tgtggctcgt 5130

gggacacagt ggtgtaggtg tgtccatttg accagaggta atgtaggggc tacatagaga 5190

ttagaaacag acaccctaaa cagagaaagc ttgttctgtg tgggacacat ctcaaggaca 5250

gaagctcttt gaaactctga caacagacat tgtcaaggtc aggtgaggag gtaggagtga 5310

tttcttagag tagtaattga aactcggtgt gggtcgggcc cacactgatg gctgaggctg 5370

ctgctggcgc ctggtggacg gtgcttatct tgtgtctatg aagtcactgt gactaaccag 5430

gtttgtccag ggacaaatat ttggcccctg gctagaacac acctgtggct agggaacaca 5490

gccatggtat gcctgtctag gaagccccat cttcaaccta gtgtggggag cagaggggag 5550

ccactccacc cccctgggat ggggtggaag actcaagcag cattcttgct ttcca gag 5608

Lys

1

gca cac cca gta ttc cat cca tcg cag tcc gac aca tac acg gag gta 5656

Ala His Pro Val Phe His Pro Ser Gln Ser Asp Thr Tyr Thr Glu Val

5 10 15

ggg aat cat ttc tcc atc cag tat ggt acc ggg agc ctg aca gga atc 5704

Gly Asn His Phe Ser Ile Gln Tyr Gly Thr Gly Ser Leu Thr Gly Ile

20 25 30

att gga gct gat caa gtc tct gtgag tacaatgccc acatcttgcc ccagaactag 5760

Ile Gly Ala Asp Gln Val Ser

35 40

gcctggggat agcaggatat gattcaaagc ctcaggggca cagtttcagc tctgccttgg 5820

gacgagaaga caaggttatt tctagcacaa gggaacagct cgttctacct gagatagccc 5880

ggtggccatc tttgcaggat gctgaaccta gagagtagtg tggggcttcc ttcagggaac 5940

aagggtcagt cagggcatga tgccttgtgt cagggctgga tcaggaaacc ctgggaactg 6000

aggggataag aacaggctgc catcttgaaa gaaaaaagac gttgaagccc gtagacaggc 6060

tggggctcca ttagtggagt gggaaggcag gaggcaggaa tccaagacag ggaagaagcc 6120

aggtatcaaa gccccaagtt ccttgaactt ctcatggcca gaggtcccga atgtccctga 6180

tttgtaggaa tcagagagtc aggagggccc tggtcctgcc tggatacttt atgcaagtgt 6240

atagtcccag ggcttggtag aaatagagaa tgggccaccc ggatgggtga gtattattct 6300

cgatctagcc tgatccaaag gccagcaaag cttagtcttc atcagcatct cttccaaact 6360

ttgaacatgg gatatatgat taactgaaaa agggctgcta acagaaatct ctcttttctt 6420

ctctgtcccc ctcctcacat taaacctggt gcatatcttt ctgggccctt atgacgtgtg 6480

tggggtcgga gtactgtagt gagctggtca tcaaatagta taaagcaaag caaatatgtc 6540

aatggcctta attattctct ttagggtagt aggtctcctt aaaatatgtc taagtgggtt 6600

ggatagatgg ctcagtggtt aagagcacag actgctcttc caaaagaccc cggttcaatc 6660

cccagtaccc aatgtcagct cgtatctgtc tgtagctcca gtcccagcag agccaggact 6720

cagacacaag tgcaggaaaa caccaatgca aagaaagaga agccaataca agaaaaagaa 6780

atcactaaag acaatttaaa aaaacaattt aagtaaagcc agctatggtg gcacacacct 6840

ttaatcccag aacttagaaa gctgcagcca atggatctct gagttcgagg ccagcctagc 6900

ctacatagag agttccaggc cagctaagga ctacatagtg aaaccctgtc tccaaacttg 6960

aaaaagtttc agtaatgtct tcttatcata aaaatgcttt ttatgtaaaa ttatataaat 7020

gaagcagagc tatcagttga aattttacat gcatatgtaa atacacatta tgtgtatatg 7080

ttacagtata aataaccact tggaaggctg catgtcaacc tgatcattgt agaaagtctg 7140

gagagcaggg aggagactga gatctacagg aggtgggctc tggcatacag cttctcgcct 7200

ctctgctagt tgccctgaac agagcagaga gcagccaagc ccatagcaac ttcagctgca 7260

atggcctctc cttcagcact tctggctctg gggcaggtgc ccatttgcct ctgtggcagt 7320

gttagcttct actgtgaagc attccttcac tgaatataga ggacatcatg gtgccagtcc 7380

atccctacag gctgcagctg gcatctttcc cacttggcac gtagcttccc tctgctgctg 7440

cttctgtaga cctcaggctg caaccctagg ttagcaacca catcctaagt gcactcccag 7500

ctggcatctg caatggtata aagccaagta cctaacacat atatagcata taagcacaca 7560

gattagtaca gttcatgcag cctgaaatgt aaagatcaga aaatcagtga tctaagccgc 7620

ggtttggagt aacaaacact aagaaacaag ctgttaataa acaggcgcaa gccaaaccca 7680

agggaaagaa atcacataga gaaggacagg gcatcggtac aataggaaat ccacactata 7740

aacatacagc caggcgtggt ggcgcacacc tttaattcca gcacttggga ggtagaggca 7800

gaggcaggca gatttctgag ttcgaggcca gcctggtcta caaagtgagt tccaggacag 7860

ccaggactac aaagagaaac cctgtctcaa aaaacataca aacaaaaaaa caaaacaaaa 7920

aaatcaacta cgtcacagat taactgtttg caaagagcaa ccaaattggt aaccctctgg 7980

cataactaat ccagaaaaat gaaggaaata caatgacctt gtcaatctca aaacaagatg 8040

tcaaaaaatg cttgatattg atacactaca ataattttaa atagatgact atcaaaactg 8100

atacaataaa atagaaatga ttaataatct ggtatctatt ttaagtggtg attctgctat 8160

taaaacaaca acaaaacaaa acaaaaaacc cacttcccac aaggaagtga cagatccatc 8220

ttccagagaa ttgatagaat tccttcataa cttcaccaga acaactatga taacaaaatg 8280

tgaaaagatt cttatacaca ctgagacttg ctagacatag tggcacaggc ctttaatccc 8340

tgtactcagg gggcagaggc aggcagacct ctatgaatgt caggccagtc tggtccacat 8400

agagaatttt aggactgcca agactatgta gtgagaccat gtctcaacca gtcgcccagt 8460

gcagtgagca gagtgcttgt ctagtgtacg caaagcccag aattgcatcc ccaggaccac 8520

ataaactgga caaggtgata catatgtata atcccagcac ccaggaggca gaggcaagag 8580

ggtcagaagt tgaagatttc ctccattgca tagtatgctc agtgctagcc tgggctatat 8640

gagatcctgt aggaatatgt aatgtgttca ccagaggaag tgccagtgca ccattattga 8700

taatcaaaca ttggaaacag cccagctatc cacccatagt ataaggcagt gtttctcaac 8760

ctgtgggtag caacctcttt ggatcaattt gtaacaatga aaatataagc cacacacaca 8820

cacacacaca cacacacaca cacacacaca cacacacatt atatatatac atatataatg 8880

taatatatgt gtgtgtattt acattatgat ccacaatagt agcaaaatta cagttacaaa 8940

gtagcagcga aaatgtcatg gcgggtggtc atcacaacat gagaaactgt attaaaggtt 9000

gccccagtag taggaaggct gatgacccca aaaataggca aaaccaatgt tgcttgagag 9060

atgagtcact cttgagggat acaaagactg gcagggtata tgaaaggcct gctggggctt 9120

cggattgtcc tgtcttattt cagaagggtt acataagaat gtggactgtt attaaaaaat 9180

agacactcat gttttctgca ttacattata ttttgatttt taaagtcaca tgaagaaagt 9240

ccaagactgg gattatagtg aaaatctgtg tgcctatttc cccaggaggc tgagaaagga 9300

agatctagct tcacccggga atctgagacc ctccttcaac taacctttgt gtaattctcc 9360

cttcaggtgg aagggttgac t gtg gat ggc cag cag ttt gga gaa agt gtc 9411

Val Glu Gly Leu Thr Val Asp Gly Gln Gln
1 5 10
aag gag cca ggc cag acc ttt gtg aat gca gag ttt gat ggg att ctg 9459
Phe Gly Glu Ser Val Lys Glu Pro Gly Gln Thr Phe Val Asn Ala Glu
15 20 25
ggt ctg gga tac ccc tca ttg gct gct gga gga gtg acc cca gtg ttt 9507
Phe Asp Gly Ile Leu Gly Leu Gly Tyr Pro Ser Leu Ala Ala Gly Gly
30 35 40
gac aac atg atg gcc cag aac ctt gtg gct ctg cct atg ttt tct gtc 9555
Val Thr Pro Val Phe Asp Asn Met Met Ala Gln Asn Leu Val Ala Leu
45 50 55
tac ttg agc agg tga ggc cag tca a gtcactgagg tccaatcagt gaatgactac 9610
Pro Met Phe Ser Val Tyr Leu Ser
60 65
aggcagatgg cctggtacat aagacttcaa ggttctgagg cccatgaatc tctcgaatct 9670
cttccttggc ctatgatgta tatgatcttg agaaaaaaac aaaaacaaaa acaaaaacaa 9730
aaacaaaaaa accctgaagc aatttggatt tcaaggaaat ggggtttgac tcataaaaga 9790
gttgagttga gcactttaca atctagcacg gtgagttaaa gttatagacc gttgcgattg 9850
gctagtgatt tatttggtca ttaaattcta aggtcctttt atatgctaag tgtgacagta 9910
gacaccaaaa cacaaggaaa caagagtagt ctccgtggtt gggagagaag gaaaagatta 9970
accagcaaga tgatactaaa agtaacttct acattagggc gaggaaagga aactgtagta 10030
tagccatgta gtagaatatc acttggtctc tggaaggaaa accagtgagg gatgcagtgt 10090
gcagtagagg acttacctag ctgacacaag gctccgggtt cactctccaa tgcctcacag 10150
acatgtgcta gaacatggat tatgctaaat gaactaagcc agtagcccaa aggacagata 10210
caaattatac tttatgcgag gtacctagaa ctgaattcat aggcgaaaag tgcagtggtg 10270
gttgcgacgg ggtagaggga taaataggaa gggggttctc gcttaatgag tctcagttct 10330

acactgtgaa aatgttctgg agaacagctt cccaggaatg aaattctgaa gtgcacactc 10390

aaaaaatggt taagcttgtt ctatcttatg gctgtactgc cacgagtttt aaagatgcac 10450

acactacagt taaaaagagc atggagctca ccgctctagc ctggggtgct gaggaagtca 10510

ctttcctacc tacacagaaa gatttacctt tcccactctg gcctggcctg gcctgagctc 10570

accacattcc agtgtaagtc ccctttccct gtccccctcc cacatcagaa gttcctggag 10630

tgagtggctg cagctgggac caggcctggg tagggttttg attgagcccc tgctgtcaga 10690

actcctgttt cctctgcaga ggctcctgaa ctacctgcta aagaagtgtg cagctttggt 10750

cagtagcaag gccagcatag cctggaacta agtctgcatg tttgtctccc agggcctagg 10810

gttcctcagg caaatgtgag gctgctgtga gaattcaaca agatcgggtg gagtgagaat 10870

tttggtttct tcaaagacag aaatgtcagc ggaatctgtt ttcattttaa tccctggtgt 10930

ggcatatggg gctgctttag attgtccaca gcagccaact atgatttacc caatgcatgc 10990

tctagagggg gcatgatttt gccagctgca gataggttct gcaggtatat gatgtttgaa 11050

agtctggaga ctcttgagag ggtatagaag ctgctgctgg tggtggttgc tgctgatgcg 11110

gtttgtcaag tagtcacgca caaagggtca caagaaggaa aaattagata gcctgattgc 11170

taagatcgaa cttgtcccaa ggaactctat gcccctaatc agcgggaagt aatctaacaa 11230

tatcattgac ccctttcctc tcaaccttct ttctctctac ctagttctta gggtgggggc 11290

aaatggaagg gtgaaatagg ggtgaagaag agtggcagga aaaagaacct ataaagtagc 11350

caaaagttgg cttactgtca tatggttaga ttgctagaac ccggcaagca agtgttctac 11410

cccttagcta tgtcctcagt ccccaataaa cctgaaattc tctactgaca tatacttcct 11470

tccctactgc tactctatcc tgcagcaccc caggcttaga atctttcctc ctggaaatgc 11530

cctcctctcc agaatcctcc taaatcccag cataaaactt gagaaataac taccaaggaa 11590

aattagccga tagtaaagcc ccagtaagac atgttgcttt caaatgatct tttctgaaga 11650

agtggatacg gtagaaaccc cagacaccac agcatcaata gcagggaaga tactgtgaga 11710

aagagtattt tgggggcatc ctcagttgca cagtgtttgt atccaacacc caagatagtc 11770

ctgaccttag atctagagtt cagatactcc ctacctcctg aactttttat ctaagaagca 11830

ggggatatgc acagggtgtt agacagagtt gttcctatct ttgatcccca aagacgcagg 11890

ctacctaggt agtttctgcc tactttgcca ccagatgtga gtgcccggac atgcaacaaa 11950

ggggccctga actcctgtct gtgtttccag aggccatttc ttgacaacct gcctttctct 12010

ctgcatagt gac cct caa ggt ggc tca ggc agt gag ctg act ttc gga ggc 12061

Asp Pro Gln Gly Gly Ser Gly Ser Glu Leu Thr Phe Gly Gly

1 5 10

tat gac ccc tct cat ttc tct ggg agc ctc aac tgg atc cca gtc acc 12109

Tyr Asp Pro Ser His Phe Ser Gly Ser Leu Asn Trp Ile Pro Val Thr

15 20 25 30

aag caa gcc tat tgg cag att gcc ctg gat ggg tgagtatc ctctagatag 12160

Lys Gln Ala Tyr Trp Gln Ile Ala Leu Asp Gly

35 40

tagctatggt tgaatggaag agtggccaca actcaaactg gattaactgg gtgatgctgc 12220

aggacactag aacagatgct gggagcctca catctggact ggagatgtga aaacacacag 12280

gaaggacaag aggggcagct tccttctgtg tacatttagc tggcctccct ctgactcaca 12340

caaccagcta tcctgtcttc aagcagcctt aattgataca caaggcaagg gctgagcacc 12400

agggaaaaga cactggctag ctgaactcaa agcctgtatg gaatatgagg tgtggtccca 12460

aatcaccacc acacaaaatg gatgtggtca gtgttctgaa agccaccggt atggccagcc 12520

aacagcacaa gatgtctctg tctgaagact gctcatgttt tgtggaggca atgactctgg 12580

aactgagcct tgaaagccat gcctgggcat gaagcaaatg gggaagcagc attgcgtacc 12640

tgatagatat atggccagcc ccgtgctagt taaggggaca gtttaaggag aacaatctag 12700

tttctgttcc tcagatgatc tggataaaaa gtgaaagttc caactctttg tagctcctga 12760

cgcttttccg actgagattt acctatttat gctcatgaga tcgttttaag gaatctcggt 12820

ccgtttgctt ggttttattt tttgaggcag gatcttccca tgtagcccca gctgtcctag 12880

aacgctcagg atcctgtcag cctcctgcat gctggagtta caggtgtccc caccatgccc 12940

agctctccat ctgcatttct gaataaaaac agagcacagt agaaaattca agaatgtatt 13000

tcagtgtgtg tgtgttcatg tgctcgtgca tacttgatca tgaattaaat gtattcctgc 13060

cctaaatctc agtcaaaaaa gcaagttgag tcaagtataa tggctcatgc ctataattcc 13120

agcatccatg aggactgcta tgggtttgag attagcctgg acaggatgaa gatgtcttaa 13180

aaagaagcaa gttggccagg catagtggtg catgccttta atcccagcac ctgggagaca 13240

aaggcaggtg gatctctgaa ttcaaggcca gtctatatag tgagttctag aaccaccaaa 13300

gcgatgcaga gagaccctat ctcaaacaaa caaatatgct agttgaaata agctactact 13360

cctgtggtag aagagaccaa agagaacacc attattgtga cctaatatag taaagggccc 13420

cagagagaga gagagagaga gagagagaga gagagagaga gagagagaga gcaggaatgg 13480

ccaaggatca tgtgttaacc atggaccatg ggactctttg tcctcagaa atc cag gtg 13538

Ile Gln Val

1

gga gac act gtg atg ttc tgc tcc gaa ggc tgc cag gcc ata gtg gac aca 13589

Gly Asp Thr Val Met Phe Cys Ser Glu Gly Cys Gln Ala Ile Val Asp Thr

5 10 15 20

ggg acc tct ctc atc act ggc ccc ccc gac aag atc aaa cag ctt caa gag 13640

Gly Thr Ser Leu Ile Thr Gly Pro Pro Asp Lys Ile Lys Gln Leu Gln Glu

25 30 35

gcc att ggg gcc aca ccc att gat gga gaa gtgagtgtct accgagggtg 13690

Ala Ile Gly Ala Thr Pro Ile Asp Gly Glu

40 45

gaggatggta gaggagcagg agatcctgtc cttgtctggg gagagagtgg gggtctgagt 13750

tgatccttct ctgttttag tat gca gtg gat tgt gcc act ctc gac acg atg 13802

Tyr Ala Val Asp Cys Ala Thr Leu Asp Thr Met

1 5 10

cca aac gtt acc ttc ctc atc aac gag gtt tca tat acc ctc aac cca act 13853

Pro Asn Val Thr Phe Leu Ile Asn Glu Val Ser Tyr Thr Leu Asn Pro Thr

15 20 25

gac tac atc ctg ccg gt aagggctgtt tccttattct gtgagtcaca gtaccctccc 13910

Asp Tyr Ile Leu Pro

30 33

cacgtgcacc tgccacttcc cccttcaaag tcatctacat gaggctacca ctcttccaag 13970

gctcctccca gctagtccag gggatgccta tgtggcattc tctctgaaag agatgaagct 14030

gcctgcggta gaaactcctg gacttctagt ttccagttct gctatgaagc agcagctact 14090

caggtacaag acacttaagg gggatttctg atattgccat gaggaattta tcaacaaaca 14150

cattaatcct ccccactacc ggtgtgcaac tggagatgtt attatcctat tatacatggg 14210

aggaagctaa ggtcctcaga gttaaataaa tgcttcagat ctgactgctg gggttggaga 14270

gatggctcag cagttaagag cacttggatg ctccagggga ccacaattga gttctgcacc 14330

tgcatgtatg gctcacaaac catatgtaac tccagctcca ggaaatcaaa tgccctcttc 14390

tgatctctgt gggaccccct catgcatgtg cacagatatt gcacacatac acataaacgt 14450

tttttttaag atttatgaat ttattttatt tatgtaagca cactgtagct gtcttcagac 14510

acaccagaag agggcatcat gtcttactac atggttgtga accaccatgt ggttgctggg 14570

aattgaactc aggacctctg gaagagcagt cagtgctctt aacccctgag ccatctctac 14630

agctccccac acaaaaattt ttaaagagtg actttaaaat agataatgag ctgcttaagg 14690

aggggccacc attcaaatta cctttcttca aggtaaaatt cgtcttttat tgaaatgatt 14750

cccaagttct gagggtgagg tggaggtgaa agtctggaca ttttggaaac ctcaggtcat 14810

ttcccatccc ccagggccta ggttttgatg ttgtgctcct ttggcaacac actttaatgt 14870

gttgatcttt aagcagagtc ccctataaca tcaggatctg tgtcagcatc aagatgccaa 14930

ggtagaaggt gaccttcacc ttatccttcc caaagccacc cgatgtaaac agacatccaa 14990

acaaacacaa gcacttgctc caaggcatag tttcatggta aagcactcaa gctctaggga 15050

cagtctggaa cagcctctgc agacaccatg ttgagcacat accactgttg aaactgagcc 15110

ctgctctcac tctcagtctc tttgccatgc tactttggac cctaggactt aagttactaa 15170

gtcttagtag tcagaatgca gttctgcagc agaggctctc aaggacatga catcaccctc 15230

cagctgtgcc ccctctggga ttctggggga tgcccagctc ctttactctc ctacttttat 15290

ccaacaagag gttctgctgg taaccacacc ccggccctac ctgccaggtt ttaatcaggg 15350

atctcaaccc ataaaggatg ctgggatagc tttaaattgt atcccagtcc tctcacaagg 15410

ctgtggcttt cccacctctt catcagcaac tttgagtgtc agatgttggg aaggccacac 15470

ctgctgatag aactcaaatc tttgctaaac tgaaaaataa gactgaaaga caggagtggc 15530

ggtgaatgtc taccatccca gtggagcaga ggcaggaaag ggagttctag gcaacagagg 15590

ttacagaatg tctcaaaaaa aaaaaaaaaa gattggggga gtttacagtc tcttcataga 15650

acaatggatt tgtgcttgcc ttgtgtctca gagagcccac tcaaatgctt cttccccaga 15710

tgtgaagaag gaagtggtag tagctagcca tttatagaac agatattaag tcaagctccg 15770

cttaaaacta aaccagattg ggtttaaacc aatgcgctct aacgccaaac cttctgcagt 15830

aaggttctgt ttccacgttt ccagttatga actgatggta gagtatggcc gtaaaaacat 15890

acagttctca gaattgtaga acacccataa tgaagggagc aagcataccc tgtactgagc 15950

aagctggaat aggagcagga aatgagagaa agccctccaa ccacaaccgt ggcactcagc 16010

aaccatccaa ggccattgca cggccagcac caccaacagt acagctccca ggactacatc 16070

cattgtcccc tgtctgtttc ttttgtgccc tag gac ttg gtg gaa gga atg cag 16124

Asp Leu Val Glu Gly Met Gln

1 5

ttc tgt ggc agt ggc ttt caa gga ctt gac att cca cct cca gct ggg 16162

Phe Cys Gly Ser Gly Phe Gln Gly Leu Asp Ile Pro Pro Pro Ala Gly

10 15 20

ccc ctc tgg atc ctg ggg gat gtc ttc atc cga cag ttc tac tca gtc 16210

Pro Leu Trp Ile Leu Gly Asp Val Phe Ile Arg Gln Phe Tyr Ser Val

25 30 35

ttt gac cgt gga aat aac caa gtg gga ttg gcc ccc gca gtt ccc taag 16270

Phe Asp Arg Gly Asn Asn Gln Val Gly Leu Ala Pro Ala Val Pro

40 45 50

agggatgtat gcctacatat ggatgcctga tacccattta acctgttaga tacctttgta 16330

actatcaaag ccgtcatttc ccatggggtg tagccacccc agagtattca gaccaatcaa 16390

agcataagag tgcaccccac tcactgcaaa cacacacaca cacacaccac ctctaccatc 16450

accacgatga aagaagttct gtctatagtc ttcactgctt attgttgact ttctattatg 16510

gaaatctcta aacatgtaca cagtagacat gatggcaaga taaataccca cacacctctg 16570

cctcaggtca caacccatcc atgtgtggcc cagactctct atcttccatc cctctggttc 16630

cacgcctcta gatttggaag cagattctaa gcaccaggtc attttatcta atgtctaaca 16690

tccttacaaa tcagaattta aatgtctcac cctctcataa atgtggacct gtttttacag 16750

ttggtttatt tgtatcagga ttaaaaccag atccataact ggacaaaaaa aacccataaa 16810

ctgatttgat tttaaatatc tcttaagtct cttttcttct acagaatttt ccacactcag 16870

aattttgcca gttggaacaa ggcatggtga tacttgttcc aactataatc tcagcctata 16930

atcaaggcct ataatcttag cactcaggaa gctgaggcag gaggaccact ttatatcaag 16990

gctacaaagc aagctccagg ccaaccagga ccatgcagca agacttgatc tcagaaaaag 17050

acaaaggaaa agagtttgaa tggatagcag aaattgggag ctatcatttg acatggatgt 17110

ctacagccat ccttcctaga aaacagtagt tagaactaga ggccttgatt catggcaaaa 17170

acatgttgca tagatggcat tggtacttct gctgtatctc attaggagac cgctaatgct 17230

gggtgtctct ccacaggtca tgttaaggtg gatgagtgag tttaaggctg tcagccttca 17290

ccgggtaata caaaagtttc tgtcagcaga ccctgtggtc acactgaacc ttgttcgttg 17350

gttagggttt ctgcaattct gccccccccc cataaaaata accttccttc atcctccttc 17410

tgctcgatgt gaagtatatc tcatatacaa aaaaagataa ctgcctgaga ccttccttcc 17470

tgccttactt accagttgtc agaaataatg agcaggtact atggcaaagg ggaatggatt 17530

taaacctctg ctccaacctc tgcttttgat gctcaatttt caatgaccct ttaagttatt 17590

tcctgtgaaa tttcccatcc catctcatac acttccaccc tatcaggtaa cctcaactct 17650

tgtcccaaag agttctctgt gccatgacta taaagttcac tataaatgaa aatacttggt 17710

agactacctg acttgaagaa gggtaggaaa agtgcctgaa gtatgtcctg gggattccca 17770

gggagaaaat gaactggagg ccaacctcat tctggaggca ccacttgctt ccctcttcct 17830

ccctttcctc ttcgattctt ttaagacaga atctacaatc ttgttcttat aaaatgcagt 17890

aagtattcca tgaccctgga attaactcaa gttgttgata agatctgttt tttaactaaa 17950

gagagggcca gcaggatggc tcagcgagta aagatgcttg ttcccaaacc tgatgacccg 18010

agttcaattc ccagacccca catggtagaa gaagagatct gctcctccaa gttgttctcc 18070

aatatccgta ttattgctct gtggctgcgc gcgcacgcac acacacacac acacactata 18130

aactggcatc attattggta tgcgatgttt ttatttctac acagaagcaa tcatgggcta 18190

cgtattctct aacttgctga cttcctttca gaatatggaa gctaaggcag gaagtcagtg 18250

agttcaaggc cagcatgcac tacacatcaa gacactgtct ttaaaaaaaa atggaggagc 18310

tggagagaat gggggaagag ggtaaatccg ggggagatcg ag 18352

## Claims

1. A gene-altered non-human mammalian animal wherein a cathepsin E-associated gene is completely or partially altered.

2. The gene-altered non-human mammalian animal as claimed in claim 1, **characterized in that** said non-human mammalian animal is a rodent.

3. The gene-altered non-human mammalian animal as claimed in claim 1, **characterized in that** said non-human mammalian animal is a mouse.

4. A cathepsin E-associated gene or a DNA fragment thereof, **characterized in that** a cathepsin E-associated gene or a DNA fragment thereof has a base sequence or an amino acid sequence as represented by SEQ ID #1.

5. The cathepsin E-associated gene or the DNA fragment thereof as claimed in claim 4, **characterized in that** the cathepsin E-associated gene or the DNA fragment has two homologous recombination regions, a first homologous recombination region being present on the 5'-upstream side and a second homologous recombination region being present on the 3'-downstream side.

6. The cathepsin E-associated gene or the DNA fragment thereof as claimed in claim 4 or 5, **characterized in that** said first homologous recombination region is composed of a DNA fragment of approximately 1.2 kbp located in a region upstream from exon 1 of the cathepsin E-associated gene.

7. The cathepsin E-associated gene or the DNA fragment thereof as claimed in claim 4, 5 or 6, **characterized in that** a DNA fragment of said first homologous recombination region is located between a first cleavage site to be cleaved with restriction enzyme *StuI* and a second cleavage site to be cleaved with restriction enzyme *HindIII* in a region upstream from exon 1 of the cathepsin E-associated gene.

8. The cathepsin E-associated gene or the DNA fragment as claimed in any one of claims 4 to 7, **characterized in that** the DNA fragment of said first homologous recombination region has a base sequence ranging from base of base number 1, 438 to base of base number 2,656 of SEQ ID #1.

9. The cathepsin E-associated gene or the DNA fragment thereof as claimed in claim 4 or 5, **characterized in that** said second homologous recombination region is composed of a DNA fragment of approximately 7.0 kbp located in a region downstream from exon 4 of the cathepsin E-associated gene.

10. The cathepsin E-associated gene or the DNA fragment thereof as claimed in claim 4, 5 or 9, **characterized in that** a DNA fragment of said second homologous recombination region is located in a region on the downstream side of exon of the cathepsin E-associated gene between a position upstream by 76 bp from a third cleavage site to be cleaved with restriction enzyme *ScaI* and a position downstream by 52 bp from a fourth cleavage site to be cleaved with restriction enzyme *HpaI*.

11. The cathepsin E-associated gene or the DNA fragment as claimed in claim 4, 5, 9 or 10, **characterized in that** the DNA fragment of said second homologous recombination region has a base sequence ranging from base of base number 6,417 to base of base number 13,548 of SEQ ID #1.

12. A targeting vector, **characterized in that** a DNA fragment of said first homologous recombination region upstream from exon 1 of a cathepsin E-associated gene and a DNA fragment of said second homologous recombination region downstream from exon 4 of the cathepsin E-associated gene are inserted.

13. The targeting vector as claimed in claim 12, **characterized in that** a base sequence of the DNA fragment of said first homologous recombination region is shorter than a base sequence of the DNA fragment of said second homologous recombination region.

14. The targeting vector as claimed in claim 12 or 13, **characterized in that** the DNA fragment of said first homologous recombination region is linked to the 5'-side of a first DNA region containing a positive selection marker gene and the DNA fragment of said second homologous recombination region is linked to the 3'-side of the first DNA region containing the positive selection marker gene and to the 5'-side of a second DNA fragment region containing a negative selection marker gene.

15. The targeting vector as claimed in claim 14, **characterized in that** said positive selection marker gene is linked to a promoter.

16. The targeting vector as claimed in claim 14 or 15, **characterized in that** said promoter is PGK promoter.

17. The targeting vector as claimed in claim 14, **characterized in that** said negative selection marker gene is linked to a promoter.

18. The targeting vector as claimed in claim 14 or 17, **characterized in that** said promoter is PGK promoter.

19. The targeting vector as claimed in any one of claims 12 to 18, **characterized in that** the DNA fragment of said first homologous recombination region has a base sequence ranging from base of base number 1,438 to base of base number 2,656 of SEQ ID #1.

20. The targeting vector as claimed in any one of claims 12 to 19, **characterized in that** the DNA fragment of said first homologous recombination region has a DNA fragment of approximately 1.2 kb.

21. The targeting vector as claimed in any one of claims 12 to 20, **characterized in that** the DNA fragment of said first homologous recombination region has a DNA fragment located in a region upstream from exon 1 of the cathepsin E-associated gene between a first cleavage site to be cleaved with restriction enzyme *StuI* and a second cleavage site to be cleaved with restriction enzyme *HindIII*.

22. The targeting vector as claimed in any one of claims 12 to 21, **characterized in that** the DNA fragment of said second homologous recombination region has a base sequence ranging from base of base number 6,417 to base of base number 13,548 of SEQ ID #1.

23. The targeting vector as claimed in any one of claims 12 to 22, **characterized in that** the DNA fragment of said second homologous recombination region has a sequence of approximately 7.0 kbp.

24. The targeting vector as claimed in any one of claims 12 to 23, **characterized in that** the DNA fragment of said second homologous recombination region is a DNA fragment located in a region downstream of exon 4 of the cathepsin E-associated gene between a position upstream by 76 bp from a third cleavage site to be cleaved with restriction enzyme *ScaI* and a position downstream by 52 bp from a fourth cleavage site to be cleaved with restriction enzyme *HpaI*.

25. The targeting vector as claimed in any one of claims 12 to 24, **characterized in that** the DNA fragment of said second homologous recombination region contains exon 5 and exon 6.

26. The targeting vector as claimed in any one of claims 12 to 25, **characterized in that** the DNA fragment of said first homologous recombination region and the DNA fragment of said second homologous recombination region are inserted into a respectively predetermined position of a plasmid.

27. The targeting vector as claimed in any one of claims 12 to 26, **characterized in that** said positive selection marker gene is neomycin transferase gene and said negative selection marker gene is thymidine kinase gene.

28. A method for the production of a cathepsin E-associated gene-altered non-human mammalian animal, **characterized in that** said gene-altered non-human mammalian animal with the cathepsin E-associated gene deleted is produced by performing homologous recombination of the cathepsin E-associated gene.

29. The method for the production of the gene-altered non-human mammalian animal as claimed in claim 28, **characterized in that** said gene-associated non-human mammalian animal is a mouse.

30. The method for the production of the gene-altered non-human mammalian animal as claimed in claim 28 or 29, **characterized in that** said gene-altered non-human mammalian animal is produced by performing homologous recombination with said targeting vector as claimed in any one of claims 9 to 20.

31. The method for the production of the gene-altered non-human mammalian animal as claimed in any one of claims 28 to 30, **characterized in that**:
a cloning step of collecting a genomic clone of the cathepsin E-associated gene by cloning from a genome library of an animal species identical to said gene-altered non-human mammalian animal using a genomic DNA isolated from the animal species or a cDNA thereof as a probe;
a targeting vector-constructing step of constructing a targeting vector with an agent-resistant gene introduced thereinto by inserting a DNA fragment of a first homologous recombination region and a DNA fragment of a second homologous recombination region into a cloning vector, each of the DNA fragment thereof being obtained each by cleavage of a genomic DNA of the cathepsin E-associated gene linked to the probe with a restriction enzyme;
a homologous recombination step for obtaining a homologous recombinant by causing an occurrence of homologous recombination by introducing said targeting vector into an ES cell;
a homologous recombinant-screening step for screening the resultant homologous recombinant with the agent-resistant gene to obtain a cathepsin E gene-deleted ES cell;
an ES cell-injecting step of injecting the cathepsin E gene-deleted ES cell into an embryo;
a chimeric non-human mammalian animal-breeding step for breeding a chimeric non-human mammalian animal by implanting the embryo with the cathepsin E gene-deleted ES cell injected thereinto to a foster mother; and
a cathepsin E gene-deleted non-human mammalian animal-breeding step for breeding a cathepsin E gene-deleted non-human mammalian animal by mating the chimeric non-human mammalian animals.

32. The method for the production of the gene-altered non-human mammalian animal as claimed in claim 31, **characterized in that** said gene-altered non-human mammalian animal is a mouse.

33. The method for the production of the gene-altered non-human mammalian animal as claimed in any one of claims 28 to 32, **characterized in that** said cloning step comprises collecting said genomic clone from the cathepsin E-associated gene or the DNA fragment thereof having a base sequence listed as SEQ ID #1.

34. The method for the production of the gene-altered non-human mammalian animal as claimed in any one of claims 28 to 33, **characterized in that**, in said cloning step, said probe is a genomic DNA isolated from an animal species identical to the cathepsin E gene-deleted non-human mammalian animal or a cDNA thereof.

35. The method for the production of the gene-altered non-human mammalian animal as claimed in any one of claims 28 to 34, **characterized in that** the DNA fragment of said first homologous recombination region is a DNA fragment of approximately 1.2 kbp located on the upstream side of exon 1 of the cathepsin E-associated gene.

36. The method for the production of the gene-altered non-human mammalian animal as claimed in any one of claims 28 to 35, **characterized in that** the DNA fragment of said first homologous recombination region is a DNA fragment having a base sequence ranging from base of base number 1,438 to base of base number 2,656 of SEQ ID #1.

37. The method for the production of the gene-altered non-human mammalian animal as claimed in any one of claims 28 to 36, **characterized in that** the DNA fragment of said second homologous recombination region is a DNA fragment of approximately 7.0 kbp located on the downstream side of exon 4 of the cathepsin E-associated gene.

38. The method for the production of the gene-altered non-human mammalian animal as claimed in any one of claims 28 to 37, **characterized in that** the DNA fragment of said second homologous recombination region is a DNA fragment having a base sequence ranging from base of base number 6,417 to base of base number 13,548 of SEQ ID #1.

39. The method for the production of the gene-altered non-human mammalian animal as claimed in any one of claims 30 to 39, **characterized in that** said targeting vector is constructed by inserting or replacing the DNA fragment of the first homologous recombination region on the upstream side of exon 1 of the cathepsin E-associated gene and by inserting or replacing the DNA fragment of the second homologous recombination region on the downstream side of exon 4 of the cathepsin E-associated gene.

40. The method for the production of the gene-altered non-human mammalian animal as claimed in any one of claims 30 to 39, **characterized in that** said targeting vector is constructed by linking the DNA fragment of said first homologous recombination region to the 3'-side of a first DNA region containing a positive selection marker gene and linking the DNA fragment of said second homologous recombination region to the 5'-side of a second DNA fragment region containing a negative selection marker gene.

41. The method for the production of the gene-altered non-human mammalian animal as claimed in claim 40, **characterized in that** said positive selection marker gene is linked to a promoter.

42. The method for the production of the gene-altered non-human mammalian animal as claimed in claim 40 or 41, **characterized in that** said promoter is PGK promoter.

43. The method for the production of the gene-altered non-human mammalian animal as claimed in claim 40, **characterized in that** said negative selection marker gene is linked to a promoter.

44. The method for the production of the gene-altered non-human mammalian animal as claimed in claim 40 or 43, **characterized in that** said promoter is PGK promoter.

45. The method for the production of the gene-altered non-human mammalian animal as claimed in any one of claims 30 to 44, **characterized in that** said targeting vector contains the DNA fragment of said first homologous recombination region having a base sequence ranging from base of base number 1,438 to base of base number 2, 656 of SEQ ID #1.

46. The method for the production of the gene-altered non-human mammalian animal as claimed in any one of claims 30 to 45, **characterized in that** said targeting vector contains the DNA fragment of said first homologous recombination region having a sequence of approximately 1.2 kbp.

47. The method for the production of the gene-altered non-human mammalian animal as claimed in any one of claims 30 to 46, **characterized in that** said targeting vector has the DNA fragment of said first homologous recombination region located in a region on the upstream side of exon 1 of the cathepsin E-associated gene between a position upstream by 76 bp from a first cleavage site to be cleaved with restriction enzyme *ScaI* and a position downstream by 52 bp from a second cleavage site to be cleaved with restriction enzyme *HindIII*.

48. The method for the production of the gene-altered non-human mammalian animal as claimed in any one of claims 30 to 47, **characterized in that** said targeting vector contains the DNA fragment of said second homologous recombination region having a base sequence ranging from base of base number 5,417 to base of base number 13,548 of SEQ ID #1.

49. The method for the production of the gene-altered non-human mammalian animal as claimed in any one of claims 30 to 48, **characterized in that** said targeting vector contains the DNA fragment of said second homologous recombination region having a sequence of approximately 7.0 kbp.

50. The method for the production of the gene-altered non-human mammalian animal as claimed in any one of claims 30 to 49, **characterized in that** said targeting vector contains the DNA fragment on the downstream side of exon 4 of the cathepsin E-associated gene located in a region between a third cleavage site to be cleaved with restriction enzyme *StuI* and a fourth cleavage site to be cleaved with restriction enzyme *HpaI*.

51. The method for the production of the gene-altered non-human mammalian animal as claimed in any one of claims 30 to 50, **characterized in that** said targeting vector has the DNA fragment of said second homologous recombination region containing exon 5 and exon 6.

52. The method for the production of the gene-altered non-human mammalian animal as claimed in any one of claims 30 to 51, **characterized in that** said targeting vector is constructed by replacing or inserting the DNA fragment of said first homologous recombination region and the DNA fragment of said second homologous recombination region into respectively predetermined positions of said targeting vector.

53. The method for the production of the gene-altered non-human mammalian animal as claimed in any one of claims 30 to 52, **characterized in that** said targeting vector contains neomycin transferase gene as said positive selection marker gene.

54. The method for the production of the gene-altered non-human mammalian animal as claimed in any one of claims 30 to 53, **characterized in that** said targeting vector contains thymidine kinase gene as said negative selection marker gene.

55. The method for the production of the gene-altered non-human mammalian animal as claimed in any one of claims 30 to 54, **characterized in that** said homologous recombination step comprises introducing said targeting vector into an embryonic stem cell (ES cell) by means of electroporation.

56. The method for the production of the gene-altered non-human mammalian animal as claimed in any one of claims 30 to 55, **characterized in that** said homologous recombinant-screening step comprises subjecting the homologous recombinant to double screening.

57. The method for the production of the gene-altered non-human mammalian animal as claimed in claim 56, **characterized in that** the double screening is carried out using saidpositive selection marker gene and said negative selection marker gene.

58. The method for the production of the gene-altered non-human mammalian animal as claimed in any one of claims 56 or 57, **characterized in that** said positive selection marker gene is neomycin transferase gene and said negative selection marker gene is thymidine kinase gene.

59. The method for the production of the gene-altered non-human mammalian animal as claimed in any one of claims 30 to 58, **characterized in that** said ES cell-injecting step comprises injecting said cathepsin E gene-deleted ES cell into the embryo by means of microinjection method.

60. The method for the production of the gene-altered non-human mammalian animal as claimed in any one of claims 30 to 59, **characterized in that** said embryo is a blastocyst.

61. The method for the production of the gene-altered non-human mammalian animal as claimed in any one of claims 30 to 60, **characterized in that** said cathepsin E gene-deleted non-human mammalian animal-breeding step comprises mating the chimeric non-human mammalian animals bred with each other to breed a heterozygous non-human mammalian animal.

62. The method for the production of the gene-altered non-human mammalian animal as claimed in claim 61, further comprising a step of confirming a heterozygous type of the cathepsin E gene from DNA of the chimeric non-human mammalian animal bred in the cathepsin E gene-deleted non-human mammalian animal-breeding step by means of PCR method.

63. A method for the construction of a targeting vector, **characterized in that** a cathepsin E-associated gene-altered non-human mammalian animal is produced by subjecting the cathepsin E-associated gene to homologous recombination using the targeting vector as described in any one of claims 9 to 24.

64. The method for the construction of the targeting vector as claimed in claim 63, **characterized in that** the targeting vector is inserted with a DNA fragment of a first homologous recombination region on the upstream side of exon 1 of the cathepsin E-associated gene and a DNA fragment of a second homologous recombination region on the downstream side of exon 4 thereof.

65. The method for the construction of the targeting vector as claimed in claim 63 or 64, **characterized in that** said targeting vector is a cyclic DNA, a plasmid or a phage.

66. A method for use of a gene-altered non-human mammalian animal, **characterized in that** the cathepsin E-associated gene-altered non-human mammalian animal as described in any one of claims 1 to 3 or the cathepsin E-associated gene-altered non-human mammalian animal produced by the method for the production of the gene-altered non-human mammalian animal as described in any one of claims 45 to 62 is used for diagnosis of a disease in which the cathepsin E-associated gene is involved.

67. The method for use of a gene-altered non-human mammalian animal as claimed in claim 66, **characterized in that** said cathepsin E-associated gene-altered non-human mammalian animal is used for diagnosis of allergy.

68. The method for use of a gene-altered non-human mammalian animal as claimed in claim 66, **characterized in that** said cathepsin E-associated gene-altered non-human mammalian animal is used for diagnosis of learning disability.

69. The method for use of a gene-altered non-human mammalian animal as claimed in claim 66, **characterized in that** said cathepsin E-associated gene-altered non-human mammalian animal is used for diagnosis of memory impairment.

70. The method for use of a gene-altered non-human mammalian animal as claimed in claim 66, **characterized in that** said cathepsin E-associated gene-altered non-human mammalian animal is used for diagnosis of acceleration of fighting episode.

71. The method for use of a gene-altered non-human mammalian animal as claimed in claim 66, **characterized in that** said cathepsin E-associated gene-altered non-human mammalian animal is used for diagnosis of stress duration.
